(19) Europäisches Patentamt
European Patent Office
Office européen des brevets

(11) EP 1 492 489 B1

(12) EUROPÄISCHE PATENTSCHRIFT

(45) Veröffentlichungstag und Bekanntmachung des Hinweises auf die Patenterteilung:
**14.09.2016 Patentblatt 2016/37**

(21) Anmeldenummer: **03745264.6**

(22) Anmeldetag: **17.03.2003**

(51) Int Cl.:
***A61Q 17/04*** *(2006.01)* ***A61K 8/90*** *(2006.01)*

(86) Internationale Anmeldenummer:
**PCT/EP2003/002776**

(87) Internationale Veröffentlichungsnummer:
**WO 2003/082221 (09.10.2003 Gazette 2003/41)**

(54) **WASSERFESTE KOSMETISCHE UND DERMATOLOGISCHE LICHTSCHUTZFORMULIERUNGEN MIT EINEM GEHALT AN POLYOXYETHYLEN-POLYDODECYLGLYKOL-BLOCKPOLYMEREN**

WATERPROOF COSMETIC AND DERMATOLOGICAL SUN PROTECTING FORMULATIONS CONTAINING POLYOXYETHYLENE-POLYDODECYLGLYCOL-BLOCK POLYMERS

FORMULATIONS COSMETIQUES ET DERMATOLOGIQUES DE PROTECTION SOLAIRE RESISTANTES A L'EAU CONTENANT DES POLYMERES SEQUENCES DE POLYOXYETHYLENE-POLYDODECYLGLYCOL

(84) Benannte Vertragsstaaten:
**AT BE BG CH CY CZ DE DK EE ES FI FR GB GR HU IE IT LI LU MC NL PT RO SE SI SK TR**

(30) Priorität: **28.03.2002 DE 10214058**

(43) Veröffentlichungstag der Anmeldung:
**05.01.2005 Patentblatt 2005/01**

(73) Patentinhaber: **Beiersdorf AG**
**20245 Hamburg (DE)**

(72) Erfinder:
- **GÖPPEL, Anja**
  **22527 Hamburg (DE)**
- **GROTELÜSCHEN, Birgit**
  **27793 Wildeshausen (DE)**
- **MANN, Tobias**
  **22175 Hamburg (DE)**

(74) Vertreter: **Rabanus, Birgit**
**Beiersdorf AG**
**Patentabteilung**
**Unnastrasse 48**
**20253 Hamburg (DE)**

(56) Entgegenhaltungen:
- **BOUTET D: "NOUVEAUX GLYCOLS COPOLYMERES UTILISES EN COSMETIQUE" PARFUMS, COSMETIQUES, AROMES, SOCIETE D'EXPANSION TECHNIQUE ET ECONOMIQUE S.A. PARIS, FR, Nr. 54, 1. Dezember 1983 (1983-12-01), Seiten 49-53, XP002057442 ISSN: 0337-3029**
- **ADAM W E: "NEUE POLYALKYLENGLYKOL-COPOLYMERE FUER DIE KOSMETIK" SOFW-JOURNAL SEIFEN, OELE, FETTE, WACHSE, VERLAG FUR CHEMISCHE INDUSTRIE, H. ZIOLKOWSKY K.G. AUGSBURG, DE, Bd. 110, Nr. 15, 1984, Seiten 427-431, XP002057441 ISSN: 0942-7694**
- **PROSERPIO G: "STABILISATION DES EMULSIONS" PARFUMS COSMETIQUES AROMES, PARIS, FR, Nr. 39, 1. Juni 1981 (1981-06-01), Seiten 71-75, XP002057440 ISSN: 0337-3029**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft die Verwendung von öllöslichen und/oder in Ölen dispergierbaren Polyoxyethylen-Polydodecylglykol-Blockpolymeren zur Erhöhung der Wasserfestigkeit von O/W-Formulierungen.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut:

> Die sogenannte UV-C-Strahlung mit einer Wellenlänge zwischen 100 und 280 nm wird von der Ozonschicht der Erdatmosphäre absorbiert und findet sich dementsprechend nicht im Sonnenspektrum. Sie ist daher ohne physiologische Bedeutung beim Sonnenbaden.

Der sogenannte UV-B-Bereich liegt zwischen 290 nm und 320 nm. UV-B-Strahlen sind wesentlich für die lang anhaltende Bräunung der Haut verantwortlich, können aber gleichzeitig ein Erythem, einen einfachen Sonnenbrand oder sogar mehr oder weniger starke Verbrennungen verursachen. Auch chronische Lichtschäden, Photodermatosen und Herpes solaris können durch UV-B-Strahlung hervorgerufen werden.

Man hat lange Zeit fälschlicherweise angenommen, dass die langwellige UV-A-Strahlung mit einer Wellenlänge zwischen 320 nm und 400 nm nur eine vernachlässigbare biologische Wirkung aufweist und dass dementsprechend die UV-B-Strahlen für die meisten

Lichtschäden .an der menschlichen Haut verantwortlich seien. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

So ist es u. a. erwiesen, daß selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z. B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Die neueren Erkenntnisse über die Wirkung der UV-A-Strahlen auf die Haut haben dazu geführt, daß man den Schutzmaßnahmen für diesen Strahlenbereich nun erhöhte Aufmerksamkeit widmet. Praktisch kein Sonnenschutzprodukt kommt mehr ohne wirksame UV-A-Filterwirkung aus, reine UV-B-Filterpräparate sind selten.

Beim Auftragen eines Sonnenschutzmittels auf die Haut können die ultravioletten Strahlen durch zwei Effekte abgeschwächt werden: zum einen durch Reflexion und Streuung der Strahlen an der Oberfläche von pulverförmigen Feststoffen (physikalischer Licht-schutz) und zum anderen durch Absorption an chemischen Substanzen (chemischer Lichtschutz). Je nachdem, welcher Wellenlängenbereich absorbiert wird, unterscheidet man zwischen UV-B-Filtern (Absorptionsbereich 280 bis 320 nm), UV-A-Filtern (Absorptionsbereich 320 bis 400 nm) und Breitbandfiltern (Absorptionsbereich 290 bis ca. 380 nm).

Zum Schutz gegen UV-B-Strahlung sind zahlreiche Verbindungen bekannt, wobei deren Absorptionsmaximum möglichst um 308 nm liegen sollte, da hier die höchste Erythemwirksamkeit der Sonnenstrahlung vorliegt. Typische UV-B-Filter sind z. B. Derivate des 3-Benzylidencamphers, der 4-Aminobenzoesäure, der Zimtsäure, der Salicylsäure, des Benzophenons sowie auch des 2-Phenylbenzimidazols.

Auch zum Schutz gegen UV-A-Strahlung sind einige Verbindungen bekannt, wie insbesondere Dibenzoylmethanderivate. Allerdings sind Dibenzoylmethanderivate in der Regel nicht photostabil, weshalb kosmetische oder dermatologische Zubereitungen mit einem Gehalt an dieser Substanz zweckmäßigerweise auch bestimmte UV-Stabilisatoren enthalten sollten. Weitere bekannte UV-A-Filtersubstanzen sind bestimmte wasserlösliche, sulfonierte UV-Filtersubstanzen, wie z. B. Phenylen-1,4-bis-(2-benzimidazyl)-3,3'-5,5'-tetrasulfonsäure und ihre Salze.

Neben den reinen UV-A- oder UV-B-Filtern gibt es Substanzen, die beide Bereiche abdecken. Zu dieser Gruppe der Breitbandfilter gehören beispielsweise unsymmetrisch substituierte s-Triazin-Verbindungen, wie z. B. das 2,4-Bis-{[4-(2-ethyl-hexyloxy)-2-hydroxy]-phenyl}-6-(4-methoxyphenyl)-1,3,5-triazin (INCI: Bis-Ethylhexyloxylphenol Methoxyphenyl Triazin), bestimmte Benzophenone, wie z. B. das 2-Hydroxy-4-methoxy-benzo-phenon (INCI: Benzophenone 3) oder das 2,2'-Methylen-bis-(6-(2H-benzotriazol-2-yl)-4-(1,1,3,3-tetramethylbutyl)-phenol) (INCI: Methylene Bis-Benzotriazolyl Tetramethylene-butylphenol).

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen. Da zur Charakterisierung einer Filtersubstanz nicht nur die Lage des Absorptionsmaximums, sondern vor allem der Absorptionsbereich wichtig ist, werden von jeder Substanz Absorptionsspektren aufgenommen. Für die Dosierung der Substanzen in den fertigen Formulierungen können die Extinktionswerte aber allenfalls eine Orientierungshilfe bieten, denn durch Wechselwirkungen mit Inhaltsstoffen der Haut oder der Hautoberfläche selbst können Unwägbarkeiten auftreten. Ferner ist in der Regel schwierig vorab abzuschätzen, wie gleichmäßig und in welcher Schichtdicke die Filtersubstanz in und auf der Hornschicht der Haut verteilt ist.

Zur Prüfung der UV-A-Schutzleistung wird üblicherweise die IPD-Methode verwendet (IPD ≡ immediate pigment darkening). Hierbei wird - ähnlich der Bestimmung des Lichtschutzfaktors - ein Wert ermittelt, der angibt, um wieviel länger die mit dem Lichtschutzmittel geschützte Haut mit UV-A-Strahlung bestrahlt werden kann, bis die gleiche Pigmentierung auftritt wie bei der ungeschützten Haut.

Die Einsatzkonzentration bekannter als Feststoff vorliegender Lichtschutzfiltersubstanzen, die insbesondere auch im UV-A-Bereich eine hohe Filterwirkung zeigen, ist allerdings häufig - gerade in Kombination mit anderen zu lösenden Substanzen - begrenzt. Es bereitet daher gewisse formulierungstechnische Schwierigkeiten, höhere Lichtschutzfaktoren bzw. UV-A-Schutzleistung zu erzielen.

Da Lichtschutzfiltersubstanzen in der Regel kostspielig sind und da manche Lichtschutzfiltersubstanzen zudem schwierig in höheren Konzentrationen in kosmetische oder dermatologische Zubereitungen einzuarbeiten sind, war es eine Aufgabe der Erfindung, auf einfache und preiswerte Weise zu Zubereitungen zu gelangen, welche bei ungewöhnlich niedrigen Konzentrationen an herkömmlichen UV-Filtersubstanzen dennoch eine akzeptable oder sogar hohe UV-A und/oder UV-B-Schutzleistung erreichen.

Auch im Bereich der Sonnenschutzformulierungen spielen Emulsionen die entscheidende Rolle. Dabei werden fließfähige oder dünnflüssige Emulsionen bevorzugt, da sie sich leichter auftragen lassen als zähflüssige Cremes.

Die meisten Sonnenschutzmittel werden in Wassernähe oder bei sportlicher Betätigung (Schwitzen) angewendet, weshalb der Wasserfestigkeit solcher Formulierungen eine besondere Bedeutung beizumessen ist. Ein wasserfestes Sonnenschutzmittel schützt den Anwender nicht nur nach dem Baden, sondern bewahrt ihn auch während des Badens vor einem Sonnenbrand. Es ist ein weitverbreiteter Irrtum, daß Wasser einen guten oder gar ausreichenden Schutz vor ultravioletter Strahlung bietet. Vielmehr haben Untersuchungen gezeigt, daß noch 1 m unter der Wasseroberfläche die Durchlässigkeit für UV-B-Strahlen bei ca. 50 % liegt. Es ist daher ratsam, daß auch Wassersportler, welche z. B. schwimmern, surfern oder schnorcheln, sowie insbesondere Kinder, die oft stundenlang am bzw. im Wasser spielen, die Haut mit einem gut haftenden, durch Wasser und Schweiß nur schwer abspülbaren Sonnenprodukt vor einer zu intensiven und übermäßigen Sonneneinstrahlung schützen.

Um hohe Lichtschutzfaktoren bei gleichzeitiger sehr guter Wasserfestigkeit zu erreichen, sind W/O-Formulierungen in der Regel von Vorteil. Allerdings weisen W/O-Emulsionen häufig unbefriedigende kosmetische Eigenschaften auf: Bei der Anwendung können sie auf der Haut einen fettigen, glänzenden und zum Teil klebrigen Eindruck hinterlassen und sich - insbesondere auf behaarter Haut - schwierig verteilen lassen.

O/W-Emulsionen hingegen wirken weniger fettend auf der Haut, sind eher mattierend und ziehen schnell in die Haut ein. Sie werden vom Verbraucher im allgemeinen als leichter und kosmetisch eleganter als W/O-Emulsionen empfunden. Da Wasser die äußere Phase ist, sind O/W-Emulsionen gewöhnlich allerdings nur bedingt wasserfest.

Um die Wasserfestigkeit kosmetischer und dermatologischer Lichtschutzzubereitungen zu verbessern, kann es von Vorteil sein, Filmbildner in die kosmetischen oder dermatologischen Zubereitungen einzuarbeiten. Nach dem Stand der Technik eignen sich hierzu z. B. Polyurethane (z. B. die Avalure® -Typen von Goodrich), Dimethicone Copolyol Polyacrylate (Silsoft Surface® von der Witco Organo Silicones Group), PVP/VA (VA = Vinylacetat) Copolymer (Luviscol VA 64 Powder der BASF), $C_{20-40}$ Carbonsäure mit Polyethylen (Performacid 350 von der Fa. New Phase Technologies) sowie Filmbildner aus der Gruppe der Polymere auf Basis von Polyvinylpyrrolidon (PVP):

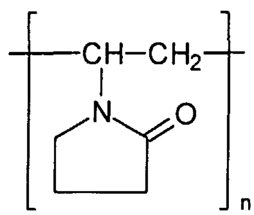

Besonders bevorzugt werden Copolymere des Polyvinylpyrrolidons eingesetzt, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind, sowie das Tricontayl PVP und dergleichen mehr.

Nachteilig ist jedoch für derartige Zubereitungen, welche Filmbildner in einer wirksamen (d. h. die Wasserfestigkeit steigernden) Konzentration enthalten, dass sie bei der Anwendung auf der Haut einen klebrigen und schmierigen Eindruck hinterlassen.

Neben dem Einfluss der Grundlage ist auch die Bindungsfähigkeit des UV-Filters in oder auf der Haut von großer Bedeutung für die Wasserfestigkeit der Formulierung. Es ist verständlich, dass öllösliche UV-Filter besser an die (lipophile) Oberfläche der Haut gebunden werden bzw. schwerer von dieser abwaschbar sind als wasserlösliche UV-Filter.

Eine weitere Aufgabe der Erfindung war es daher, auf einfache und preiswerte Weise zu Zubereitungen (insbesondere zu O/W-Formulierungen) zu gelangen, welche sich durch eine gute Wasserfestigkeit auszeichnen. Insbesondere sollten Zubereitungen gefunden werden, welche einen hohen Gehalt an wasserlöslichen UV-Filtern haben und dennoch eine sehr gute Wasserfestigkeit zeigen.

Überraschend und für den Fachmann nicht vorauszusehen war, dass die Verwendung von öllöslichen und/oder in Ölen dispergierbaren Polyoxyethylen-Polydodecylglykol-Blockpolymeren zur Verbesserung der Wasserfestigkeit von O/W-Formulierungen den Nachteilen des Standes der Technik abhelfen würde.

Es war darüber hinaus insbesondere erstaunlich, dass die Verwendung von öllöslichen und/oder in Ölen dispergierbaren Polyoxyethylen-Polydodecylglykol-Blockpolymeren zur Verbesserung der Wasserfestigkeit kosmetischer oder dermatologischer Formulierungen enthaltend mindestens eine übliche wasserlösliche UV-Filtersubstanz, den Nachteilen des Standes der Technik abhelfen würde.

Die Zubereitungen gemäß der vorliegenden Erfindung stellen in jeglicher Hinsicht überaus befriedigende Präparate dar, welche nicht auf eine eingeschränkte Rohstoffauswahl begrenzt sind. Dementsprechend eignen sie sich ganz besonders, um als Grundlage für Zubereitungsformen mit vielfältigen Anwendungszwecken zu dienen. Die Zubereitungen gemäß der vorliegenden Erfindung zeigen sehr gute sensorische und kosmetische Eigenschaften, wie beispielsweise die Verteilbarkeit auf der Haut oder das Einzugsvermögen in die Haut, und zeichen sich ferner durch eine sehr gute Lichtschutzeffektivität, eine überaus hohe UV-A- und/oder UV-B-Schutzleistung sowie durch eine ausgezeichnete Wasserfestigkeit bei gleichzeitig hervorragenden Hautpflegedaten aus. Es war insbesondere erstaunlich, daß auf übliche Filmbildner, welche nach dem Stand der Technik zur Steigerung der Wasserfestigkeit eingesetzt werden, gänzlich verzichtet werden kann.

Ganz besonders erstaunlich war es, daß die Verwendung von öllöslichen und/oder in Ölen dispergierbaren Polyoxyethylen-Polydodecylglykol-Blockpolymeren auch die UV-A-bzw. UV-B-Schutzleistung kosmetischer oder dermatologischer Formulierungen enthaltend mindestens eine übliche UV-Filtersubstanz verbessert, da die erfindungsgemäß verwendeten Substanzen selbst keinerlei Absorptionsleistung im UV-Bereich zeigen.

Bislang existiert zwar noch keine europaweit verbindliche Methode zur Bestimmung der Wasserfestigkeit kosmetischer oder dermatologischer Lichtschutzformulierungen, aber der Stand der Technik kennt eine Reihe von möglichen Untersuchungsmethoden: Dabei wird die zu untersuchende Zubereitung auf die Haut aufgetragen und der Lichtschutzfaktor ermittelt. Dann läßt man für eine bestimme Zeit Wasser auf die Haut einwirken und bestimmt anschließend nochmals den Lichtschutzfaktor. Dieses Grundprinzip der Messung ist bei allen Methoden gleich. Unterschiede bestehen hingegen in den Details der Versuchsanordnung wie beispielsweise der Kontaktart (Becken/Whirlpool oder Schwallbrause), der Dauer der Wassereinwirkung, der Wassertemperatur oder der verwendeten Wasserart (Leitungs-, Salz- oder Chlorwasser).

Im Sinne der vorliegenden Erfindung werden Präparate als wasserfest bezeichnet, die nach folgendem Test noch mindestens 50 % des ursprünglich vorhandenen Lichtschutzfaktors aufweisen:

- Auftrag von 2 mg/cm$^2$ der zu untersuchenden Zubereitung auf zwei Hautareale (vorzugsweise am Rücken des Probanden)
- 15- bis 30-minütiges Einwirken (Warten)
- Bestimmung des (ursprünglich vorhandenen) Lichtschutzsfaktors an einem der beiden Areale
- 2 x 20-minütiger Kontakt mit Wasser (Leitungswasser) im Whirlpool bei 25 bis 35 °C (mindestens 15 Minuten Pause zwischen den Bädern)
- 15- bis 30-minütiges Trocknen (Warten)
- Bestimmung des Lichtschutzsfaktors am anderen der beiden Areale

Das obengenannte Kriterium für die Wasserfestigkeit des getesteten Produktes ergibt sich dementsprechend aus dem Quotienten der ermittelten Lichtschutzfaktoren (LSF) vor und nach dem Einwirken von Wasser:

$$\frac{LSF_{nach}}{LSF_{vor}} \cdot 100\ \%$$

Es war insbesondere erstaunlich, daß die Zubereitungen im Sinne der vorliegenden Erfindung auch gegenüber Salzwasser (Meerwasser, Schweiß) und sogar gechlortem Wasser (Schwimmbad) wasserfest sind und dementsprechend auch als schweißresistent bzw. chlorwasserresistent bezeichnet werden können.

Erfindungsgemäße Polyoxyethylen-Polydodecylglykol-Blockpolymere sind Polymere mit folgender Struktur:

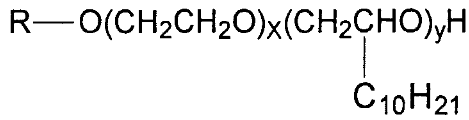

wobei R= $-CH_3$ oder $H(OC(C_{10}H_{21})HCH_2)_z$- sein kann und x, y und z Zahlen aus dem Bereich von 1 bis 100, insbesondere von 1 bis 50 darstellen und y und z gleich sind, aber unabhänig von x gewählt werden können.

Besonders vorteilhaft im Sinne der vorliegenden Erfindung sind PEG-45 Dodecylglykolcopolymer (INCI: PEG-45 Dodecyl Glycol Copolymer [y=z=11 und x=45]) und PEG-22 Dodecylglykolcopolymer (INCI: PEG-22 Dodecyl Glycol Copolymer [y=z=4,5 und x=22]) und Methoxy PEG-22 Dodecylglykolcopolymer (INCI: Methoxy PEG-22 Dodecyl Glycol Copolymer [y=7 und x=22 und R = $CH_3$]) zu verwenden, welche von der Firma AKZO Nobel erhältlich sind.

Kosmetische oder dermatologische Zubereitungen im Sinne der vorliegenden Erfindung enthalten - bezogen auf das Gesamtgewicht der Zubereitungen - vorteilhaft 0,05 bis 7 Gew.-%, insbesondere 0,1 bis 5 Gew.-% an einem oder mehreren Polyoxyethylen-Polydodecylglykol-Blockpolymeren.

Die Zubereitungen im Sinne der vorliegenden Erfindung können bevorzugt neben einer oder mehrerer Ölphasen zusätzlich eine oder mehrere Wasserphasen enthalten und beispielsweise in Form von W/O-, O/W-, W/O/W- oder O/W/O-Emulsionen vorliegen. Solche Formulierungen können vorzugsweise auch Mikroemulsionen, Stifte, Schäume (sog. Mousse), Feststoff-Emulsionen (d. h. Emulsionen, welche durch Feststoffe stabilisiert sind, z. B. Pickering-Emulsionen), sprühbare Emulsionen oder Hydrodispersionen sein. Des weiteren können die Zubereitungen vorteilhaft auch ölfreie und/oder wäßrige/alkoholische Lösungen sein.

Auch (makroskopisch) zwei- oder mehrphasige Systeme sind erfindungsgemäß vorteilhaft. "Zwei- oder mehrphasig" im Sinne der vorliegenden Erfindung bedeutet, daß zwei oder mehr Phasen separat übereinander geschichtet vorliegen. Es ist besonders vorteilhaft im Sinne der vorliegenden Erfindung, wenn mindestens eine der makroskopisch sichtbaren Phasen eine (W/O-, O/W-, Mikro-) Emulsion darstellt. Die Emulsion wird bei dieser (makroskopischen) Betrachtung als eine Phase wahrgenommen, obwohl es dem Fachmann natürlich bekannt ist, daß Emulsionen an sich aus zwei oder mehr miteinander homogenisierten Phasen gebildet werden. Die "Emulsionsphase" ist langzeitstabil, so daß es auch über einen längeren Zeitraum (Monate, Jahre) nicht zu einer Entmischung beziehungsweise Phasenauftrennung innerhalb der Emulsion kommt.
Die makroskopisch sichtbaren Phasen bzw. Schichten lassen sich vorteilhaft - zum Beispiel durch Schütteln - kurzfristig zu einer homogenen Emulsion emulgieren, welche aber nicht langzeitstabil ist, sondern sich vielmehr über einen Zeitraum von Minuten, Stunden oder Tagen wieder zu zwei oder mehr übereinander geschichteten Phasen entmischt. Es ist besonders vorteilhaft im Sinne der vorliegenden Erfindung, wenn mindestens eine der makroskopisch sichtbaren Phasen eine Mikroemulsion und mindestens eine andere der makroskopisch sichtbaren Phasen eine Ölphase darstellt.

## Sprühbare Emulsionen, insbesondere Mikroemulsionen

Besonders vorteilhaft im Sinne der vorliegenden Erfindung sind sprühbare O/W-Emulsionen, insbesondere O/W-Mikroemulsionen.

Die Tröpfchendurchmesser der gewöhnlichen "einfachen", also nichtmultiplen Emulsionen liegen im Bereich von ca 1 μm bis ca. 50 μm. Solche "Makroemulsionen" sind, ohne weitere färbende Zusätze, milchigweißgefärbt und opak. Feinere "Makroemulsionen", deren Tröpfchendurchmesser im Bereich von ca. 0,5 μm bis ca. 1 μm liegen, sind, wiederum ohne färbende Zusätze, bläulichweißgefärbt und opak. Solche "Makroemulsionen" haben für gewöhnlich ein hohe Viskosität.

Der Tröpfchendurchmesser von Mikroemulsionen im Sinne der vorliegenden Erfindung dagegen liegt im Bereich von etwa 50 bis etwa 500 nm. Derartige Mikroemulsionen sind bläulichweiß gefärbt bis transluzent und meist niedrigviskos. Die Viskosität vieler Mikroemulsionen vom O/W-Typ ist vergleichbar mit der des Wassers.

Vorteil von Mikroemulsionen ist, daß in der dispersen Phase Wirkstoffe wesentlich feiner dispers vorliegen können als in der dispersen Phase von "Makroemulsionen". Ein weiterer Vorteil ist, daß sie aufgrund ihrer niedrigen Viskosität versprühbar sind. Werden Mikroemulsionen als Kosmetika verwendet, zeichnen sich entsprechende Produkte durch hohe kosmetische Eleganz aus.

Erfindungsgemäß vorteilhaft sind insbesondere O/W-Mikroemulsionen, welche mit Hilfe der sogenannten Phaseninversionstemperatur-Technologie erhältlich sind und mindestens einen Emulgator (Emulgator A) enthalten, welcher gewählt wird aus der Gruppe der Emulgatoren mit folgenden Eigenschaften:

- ihre Lipophilie ist abhängig von der Temperatur, dergestalt daß durch Erhöhung der Temperatur die Lipophilie zunimmt und durch Senkung der Temperatur die Lipophilie des Emulgators abnimmt.

Vorteilhafte Emulgatoren A sind z. B. polyethoxilierte Fettsäuren (PEG-100 Stearat, PEG-20 Stearat, PEG-150 Laurath, PEG-8 Distearat und dergleichen) und/oder polyethoxilierte Fettalkohole (Cetearath-12, Cetearath-20, Isoceteth-20, Beheneth-20, Laureth-9 etc.) und/oder Alkylpolyglycoside (Cetearyl Glycoside, Stearyl Glycoside, Palmityl Glycoside etc.).

Sofern die Phaseninversion im wesentlichen durch Variation der Temperatur eingeleitet wird, sind O/W-Emulsionen, insbesondere O/W-Mikroemulsionen erhältlich. Die Tröpfchengrößen liegen in der Regel zwischen 20 und 500 nm.

Dabei wird die Größe der Öltröpfchen im wesentlichen durch das Konzentrationsverhältnis von eingesetzten Emulgatoren zur Ölphase bestimmt: ein höherer Emulgatoranteil bewirkt kleinere Tröpfchen und ein geringerer Emulgatoranteil führt zu größeren Tröpfchen, wobei es zu Instabilitäten der Mikroemulsionen kommen kann, wenn die Öltröpfchen zu groß werden.

Da viele UV-Filtersubstanzen öllöslich sind und daher Bestandteile der Ölphase darstellen, ist die Ölphase von kosmetischen oder dermatologischen Lichtschutzzübereitungen in der Regel größer als die vergleichbarer Zubereitungen ohne UV-Filtersubstanzen. Dies kann aber nach dem obengesagten im schlimmsten Fall zur Instabilität des Produktes führen. Diesem Phänomen läßt sich selbstverständlich entgegenwirken, indem man die Emulgatorkonzentration (deutlich) erhöht und auf diesem Wege das Emulgator-Ölphasen-Verhältnis möglichst konstant hält.

An sich ist die Verwendung der üblichen Emulgatoren in kosmetischen oder dermatologischen Zubereitungen unbedenklich. Dennoch können Emulgatoren, wie letztlich jede chemische Substanz, im Einzelfalle allergische oder auf Überempfindlichkeit des Anwenders beruhende Reaktionen hervorrufen. Insbesondere in sehr hohen Konzentrationen können Emulgatoren Unverträglichkeitsreaktionen hervorrufen, weshalb es nicht an Versuchen gefehlt hat, die Menge an üblichen Emulgatoren auf ein Minimum zu reduzieren.

Es war erstaunlich und beruht ebenfalls auf erfinderischer Tätigkeit, daß sich durch die Verwendung von öllöslichen und/oder in Ölen dispergierbaren Polyoxyethylen-Polydodecylglykol-Blockpolymeren die Tröpfchengröße der inneren Phase von Mikroemulsionen reduzieren läßt. Auf diese Weise läßt sich dementsprechend die Stabilität von Mikroemulsionen, welche UV-Filtersubstanzen enthalten, erhöhen, ohne daß die Emulgatormenge unnötig vergrößert werden müßte. Dieser Effekt läßt sich insbesondere vorteilhaft einsetzen in Mikroemulsionen, die durch Phaseninversion hergestellt wurden.

Es ist im Sinn der vorliegenden Erfindung gegebenenfalls vorteilhaft, weitere, nicht unter die Definition des Emulgators A fallende, W/O und/oder O/W-Emulgatoren zu verwenden, beispielsweise um die Wasserfestigkeit der Zubereitungen gemäß der vorliegenden Erfindung zu erhöhen. Hier können z. B. Alkylmethiconcopolyole und/oder Alkyl-Dimethiconcopolyole (insbesondere Cetyl Dimethicone Copolyol, Lauryl Methicone Copolyol), W/O-Emulgatoren (wie z. B. Sorbitanstearat, Glycerylstearat, Glycerolstearat, Sorbitanoleat, Lecithin, Glycerylisostearat, Polyglyceryl-3-Oleat, Polyglyceryl-3 Diisostearat, PEG-7-hydriertes Ricinusöl, Polyglyceryl-4-isostearat, Acrylat/ $C_{10-30}$-Alkylacrylat-Crosspolymer, Sorbitanisostearat, Poloxamer 101, Polyglyceryl-2-dipolyhydroxystearat, Poly-glyceryl-3-Diisostearat, Polyglyceryl-4-dipolyhydroxystearat, PEG-30-dipolyhydroxystearat, Diisostearoylpolyglyceryl-3-diisostearat, Glycol Distearat, Polyglyceryl-3-dipolyhydroxystearat) und/oder Fettsäureester der Schwefel- oder Phosphorsäure (Cetylphosphat, Trilaureth-4 Phosphat, Trioleth-8-Phosphat, Stearylphosphat, Cetearylsulfat etc.) verwendet werden.

Weitere vorteilhafte sprühbare O/W-Emulsionen im Sinne der vorliegenden Erfindung sind dünnflüssige kosmetische oder dermatologische Hydrodispersionen, welche mindestens eine Ölphase und mindestens eine Wasserphase enthalten, wobei die Zubereitung durch mindestens einen Gelbildner stabilisiert wird und nicht notwendigerweise Emulgatoren enthalten muß, aber einen oder mehrere Emulgatoren enthalten kann.

Vorteilhafte Gelbildner für derartige Zubereitungen sind beispielsweise Copolymere aus $C_{10-30}$-Alkylacrylaten und einem oder mehreren Monomeren der Acrylsäure, der Methacrylsäure oder deren Ester. Die INCI-Bezeichnung für solche Verbindungen ist "Acrylates/C 10-30 Alkyl Acrylate Crosspolymer". Insbesondere vorteilhaft sind die Pemulen® Typen TR 1, TR 2 und TRZ von der Fa. Goodrich (Noveon).

Auch Carbopole sind vorteilhafte Gelbildner für derartige Zubereitungen. Carbopole sind Polymere der Acrylsäure, insbesondere auch Acrylat-Alkylacrylat-Copolymere. Vorteilhafte Carbopole sind beispielsweise die Typen 907, 910, 934, 940, 941, 951, 954, 980, 981, 1342, 1382, 2984 und 5984. ebenso die ETD-Typen 2020, 2050 und Carbopol Ultrez 10. Weitere vorteilhafte Gelbildner für derartige Zubereitungen sind Xanthan Gummi, Cellulose Derivate und Johannisbrotkernmehl.

Als mögliche (fakultative) Emulgatoren können ethoxilierte Fettalkohole oder ethoxilierte Fettsäuren (insbesondere PEG-100 Stearat, Ceteareth-20) und/oder andere nichtionische oberflächenaktive Substanzen verwendet werden.

Ferner vorteilhaft können die sehr dünnflüssigen bis sprühbaren Emulsionen auch W/O- bzw. Wasser-in-Silikonöl- (W/S-) Emulsionen sein. Insbesondere vorteilhaft sind W/O- bzw. W/S-Emulsionen, die

- mindestens einen Silikonemulgator (W/S) mit einem HLB-Wert ≤ 8 und/oder mindestens einen W/O-Emulgator mit einem HLB-Wert < 7 und
- mindestens einen O/W-Emulgator mit einem HLB-Wert > 10 enthalten.

Derartige Zubereitungen enthalten ferner mindestens 20 Gew.-% Lipide, wobei die Lipidphase vorteilhaft auch Silikonöle enthalten bzw. sogar ganz aus solchen bestehen kann.

Der oder die Silikonemulgatoren kann vorteilhaft aus der Gruppe der Alkylmethiconco-polyole und/oder Alkyl-Dimethiconcopolyole gewählt werden (z. B. Dimethiconcopolyole, welche von der Goldschmidt AG unter den Warenbezeichnungen ABIL® B 8842, ABIL® B 8843, ABIL® B 8847, ABIL® B 8851, ABIL® B 8852, ABIL® B 8863, ABIL® B 8873 und ABIL® B 88183 verkauft werden, Cetyl Dimethiconcopolyol [Goldschmidt AG / ABIL® EM 90], Cyclomethicon Dimethiconcopolyol [Goldschmidt AG / ABIL® EM 97], Laurylmethiconcopolyol [Dow Corning Ltd. / Dow Corning® 5200 Formulation Aid], Octyl Dimethicon Ethoxy Glucosid [Firma Wacker].

Der oder die W/O-Emulgatoren mit einem HLB-Wert < 7 kann vorteilhaft aus folgender Gruppe gewählt werden: Sorbitanstearat, Sorbitanoleat, Lecithin, Glyceryllanolat, Lanolin, hydriertem Ricinusöl, Glycerylisostearat, Polyglyceryl-3-Oleat, Pentaerythrithylisostearat, Methylglucosedioleat, Methylglucosedioleat im Gemisch mit Hydroxystearat und Bienenwachs, PEG-7-hydriertes Ricinusöl, Polyglyceryl-4-isostearat, Hexyllaurat, Acrylat/ $C_{10-30}$-Alkylacrylat-Crosspolymer, Sorbitanisostearat, Polyglyceryl-2-dipolyhydroxystearat, Poly-glyceryl-3-Diisostearat, PEG-30-dipolyhydroxystearat, Diisostearoylpolyglyceryl-3-diisostearat, Polyglyceryl-3-dipolyhydroxystearat, Polyglyceryl-4-dipolyhydroxystearat, Polyglyceryl-3-dioleat.

Der oder die O/W-Emulgatoren mit einem HLB-Wert > 10 kann vorteilhaft aus folgender Gruppe gewählt werden: Glycerylstearat im Gemisch mit Ceteareth-20, Ceteareth-25, Ceteareth-6 im Gemisch mit Stearylalkohol, Cetylstearylalkohol im Gemisch mit PEG-40-Ricinusöl und Natriumcetylstearylsulfat, Triceteareth-4 Phosphat, Glycerylstearat, Natriumcetylstearylsulfat, Lecithin Trilaureth-4 Phosphat, Laureth-4 Phosphat, Stearinsäure, Propylenglycolstearat SE, PEG-9-Stearat, PEG-20 Stearat, PEG-30-Stearat, PEG-40-Stearat, PEG-100-Stearat, Ceteth-2, Ceteth-20, Polysorbate-20, Polysorbate-60, Poly-sorbate-65, Polysorbate-100, Glycerylstearat im Gemisch mit PEG-100 Stearat, Ceteareth-3, Isostearylglycerylether, Cetylstearylalkohol im Gemisch mit Natrium Cetylstearylsulfat, PEG-40-Stearat, Glycol Distearat, Polyglyceryl-2-PEG-4-Stearat, Ceteareth-12, Ceteareth-20, Ceteareth-30, Methyl-glucosesesquistearat, Steareth-10, PEG-20-Stearat, Steareth-21, Steareth-20, Isosteareth-20, PEG-45/ Dodecylglycol-Copolymer, Glycerylstearat SE, Ceteth-20, PEG-20-Methylglucosesesquistearat, Glycerylstearatcitrat, Cetylphosphat, Cetearyl Sulfat, Sorbitansesquioleat, Triceteareth-4-Phosphat, Trilaureth-4-Phosphat, Polyglyceryl-methylglucosedistearat, Kaliumcetylphosphat, Isosteareth-10, Polyglyceryl-2-sesquüsostearat, Ceteth-10, Isoceteth-20, Glycerylstearat im Gemisch mit Ceteareth-20, Ceteareth-12, Cetylstearylalkohol und Cetylpalmitat, PEG-30-Stearat, PEG-40-Stearat, PEG-100-Stearat.

Ferner vorteilhaft sind wäßrig-alkoholische Lösungen. Sie können von 0 Gew.-% bis 90 Gew.-% Ethanol enthalten. Wäßrig-alkoholische Lösungen können im Sinne der vorliegenden Erfindung vorteilhaft auch Lösungsvermittler enthalten, wie z. B. PEG-40 oder PEG-60 hydrogeniertes Ricinusöl.

Die Zubereitungen gemäß der vorliegenden Erfindung können vorteilhaft auch als kosmetische oder dermatologische Tränkungslösungen, mit welchen insbesondere wasserunlösliche Substrate - wie z. B. gewebte oder nichtgewebte Tücher - befeuchtet sind, verwendet werden. Derartige Tränkungslösungen sind vorzugsweise dünnflüssig, insbesondere sprühbar (wie z. B. PIT-Emulsionen, Hydrodispersionen, W/O-Emulsionen, Öle, wäßrige Lösungen etc.) und haben vorzugsweise eine Viskosität von weniger als 2000 mPa·s, insbesondere weniger als 1.500 mPa·s (Meßgerät: Haake Viskotester VT-02 bei 25 °C). Mit ihrer Hilfe sind beispielsweise kosmetische Sonnenschutztücher, Pflegetücher und dergleichen erhältlich, welche die Kombination eines weichen, wasserunlöslichen Materials mit der dünnflüssigen kosmetischen und dermatologischen Tränkungslösung darstellen.

**Öle**

Die Zubereitungen gemäß der vorliegenden Erfindung können vorteilhaft auch als wasserfreie Öle oder Ölgele oder Pasten vorliegen. Vorteilhafte Öle sind z. B. synthetische, halbsynthetische oder natürliche Öle wie beispielsweise Rapsöl, Reisöl, Avocadoöl, Olivenöl, Mineralöl, Cocoglyceride, Butylene Gylcol Dicaprylat/Dicaprat, $C_{12-15}$ Alkylbenzoat, Dicaprylyl Carbonat, Octyldodekanol und dergleichen mehr. Als Ölgelbildner können verschiedenste Wachse mit einem Schmelzpunkt > 25°C verwendet werden. Ferner vorteilhaft sind Gelbildner aus der Gruppe der Aerosile, der Alkylgalaktomannane (z. B. N-Hance AG 200 und N-Hance AG 50 von der Fa. Hercules) und Polyethylene-Derivate.

**Schäume**

Besonders vorteilhaft im Sinne der vorliegenden Erfindung sind ferner selbstschäumende, schaumförmige, nachschäumende oder schäumbare kosmetische und dermatologische Zubereitungen.

Unter "selbstschäumend", "schaumförmig", "nachschäumend" bzw. "schäumbar" sind Zubereitungen zu verstehen, aus welchen Schäume - sei es bereits während des Herstellprozesses, sei es bei der Anwendung durch den Verbraucher oder auf andere Weise - durch Eintrag eines oder mehrerer Gase im Prinzip herstellbar sind. In derartigen Schäumen liegen die Gasbläschen (beliebig) verteilt in einer (oder mehreren) flüssigen Phase(n) vor, wobei die (aufgeschäumten) Zubereitungen makroskopisch nicht notwendigerweise das Aussehen eines Schaumes haben müssen. Erfindungsgemäße (aufgeschäumte) kosmetische oder dermatologische Zubereitungen (im folgenden der Einfachheit halber auch als Schäume bezeichnet) können z. B. makroskopisch sichtbar dispergierte Systeme aus in Flüssigkeiten dispergierten Gasen darstellen. Der Schaumcharakter kann aber beispielsweise auch erst unter einem (Licht-) Mikroskop sichtbar werden. Darüber hinaus sind erfindungsgemäße Schäume - insbesondere dann, wenn die Gasbläschen zu klein sind, um unter einem Lichtmikroskop erkannt zu werden - auch an der starken Volumenzunahme des Systems erkennbar.

Deratige Zubereitungen enthalten im Sinne der vorliegenden Erfindung vorteilhaft ein Emulgatorsystem, welches aus

A. mindestens einem Emulgator A, gewählt aus der Gruppe der ganz-, teil- oder nicht neutralisierten, verzweigten und/oder unverzweigten, gesättigten und/oder ungesättigten Fettsäuren mit einer Kettenlänge von 10 bis 40 Kohlenstoffatomen,
B. mindestens einem Emulgator B, gewählt aus der Gruppe der polyethoxylierten Fettsäureester mit einer Kettenlänge von 10 bis 40 Kohlenstoffatomen und mit einem Ethoxylierungsgrad von 5 bis 100 und
C. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen besteht.

Der oder die Emulgatoren A werden vorzugsweise gewählt aus der Gruppe der Fettsäuren, welche ganz oder teilweise mit üblichen Alkalien (wie z. B. Natrium- und/oder Kaliumhydroxid, Natrium- und/oder Kaliumcarbonat sowie Mono- und/oder Triethanolamin) neutralisiert sind. Besonders vorteilhaft sind beispielsweise Stearinsäure und Stearate, Isostearinsäure und Isostearate, Palmitinsäure und Palmitate sowie Myristinsäure und Myristate.

Der oder die Emulgatoren B werden vorzugsweise gewählt aus der folgenden Gruppe: PEG-9-Stearat, PEG-8-Distearat, PEG-20-Stearat, PEG-8 Stearat, PEG-8-Oleat, PEG-25-Glyceryltrioleat, PEG-40-Sorbitanlanolat, PEG-15-Glycerylricinoleat, PEG-20-Glycerylstearat, PEG-20-Glycerylisostearat, PEG-20-Glyceryloleat, PEG-20-Stearat, PEG-20-Methylglucosesesquistearat, PEG-30-Glycerylisostearat, PEG-20-Glyceryllaurat, PEG-30-Stearat, PEG-30-Glycerylstearat, PEG-40-Stearat, PEG-30-Glyceryllaurat, PEG-50-Stearat, PEG-100-Stearat, PEG-150-Laurat. Besonders vorteilhaft sind beispielsweise polyethoxylierte Stearinsäureester.

Der oder die Coemulgatoren C werden erfindungsgemäß vorzugsweise aus der folgenden Gruppe gewählt: Behenylalkohol ($C_{22}H_{45}OH$), Cetearylalkohol [eine Mischung aus Cetylalkohol ($C_{16}H_{33}OH$) und Stearylalkohol ($C_{18}H_{37}OH$)], Lanolinalkohole (Wollwachsalkohole, die die unverseifbare Alkoholfraktion des Wollwachses darstellen, die nach der Verseifung von Wollwachs erhalten wird). Besonders bevorzugt sind Cetyl- und Cetylstearylalkohol.

Es ist erfindungsgemäß vorteilhaft, die Gewichtsverhältnisse von Emulgator A zu Emulgator B zu Coemulgator C (A : B : C) wie a : b : c zu wählen, wobei a, b und c unabhängig voneinander rationale Zahlen von 1 bis 5, bevorzugt von 1 bis 3 darstellen können. Insbesondere bevorzugt ist ein Gewichtsverhältnis von etwa 1 : 1 : 1.

Es ist vorteilhaft im Sinne der vorliegenden Erfindung, die Gesamtmenge der Emulgatoren A und B und des Coemulgators C aus dem Bereich von 2 bis 20 Gew.-%, vorteilhaft von 5 bis 15 Gew.-%, insbesondere von 7 bis 13 Gew.-%, jeweils bezogen auf das Gesamtgewicht der Formulierung, zu wählen.

**Pickering- / feststoffstabilisierte Emulsionen**

Besonders vorteilhaft im Sinne der vorliegenden Erfindung sind ferner kosmetische oder dermatologische Zubereitungen, welche nur durch feinstverteilte Feststoffteilchen stabilisiert sind. Solche "emulgatorfreien" Emulsionen werden auch als Pickering-Emulsionen bezeichnet.

In Pickering-Emulsionen kommt es zu einer Anreicherung des festen Stoffes an der Phasengrenze Öl/Wasser in Form einer Schicht, wodurch ein Zusammenfließen der dispersen Phasen verhindert wird. Von wesentlicher Bedeutung sind dabei insbesondere die Oberflächeneigenschaften der Feststoffpartikel, welche sowohl hydrophile als auch lipophile Eigenschaften zeigen sollten.

Vorteilhaft können die stabilisierenden Feststoffteilchen auch oberflächlich wasserabweisend behandelt ("gecoatet") sein, wobei ein amphiphiler Charakter dieser Feststoffteilchen gebildet werden bzw. erhalten bleiben soll. Die Oberflächenbehandlung kann darin bestehen, daß die Feststoffteilchen nach an sich bekannten Verfahren mit einer dünnen hydrophoben bzw. hydrophilen Schicht versehen werden.

Der mittlere Partikeldurchmesser der als Stabilisator verwendeten mikrofeinen Feststoffteilchen wird vorzugsweise kleiner als 100 $\mu$m, besonders vorteilhaft kleiner als 50 $\mu$m gewählt. Dabei ist es im wesentlichen unerheblich, in welcher Form (Plättchen, Stäbchen, Kügelchen etc.) bzw. Modifikation die verwendeten Feststoffteilchen vorliegen.

Vorzugsweise werden die mikrofeinen Feststoffteilchen aus der Gruppe der amphiphilen Metalloxidpigmente gewählt. Vorteilhaft sind insbesondere:

- Titandioxide (gecoatet und ungecoatet): z. B. Eusolex T-2000 von der Fa. Merck, Titandioxid MT-100 Z von der Fa. Tayca Corporation
- Zinkoxide z. B. Z-Cote und Z-Cote HP1 von der BASF AG, MZ -300, MZ -500 und MZ-505M von der Fa. Tayca Corporation
- Eisenoxide

Des weiteren ist es vorteilhaft, wenn die mikrofeinen Feststoffteilchen aus der folgenden Gruppe gewählt werden: Bornitride, Stärkederivate (Tapioca Starch, Sodium Corn Starch Octynylsuccinat etc.), Talkum, Latexpartikel.

Es ist erfindungsgemäß vorteilhaft, wenn die feststoffstabilisierten Emulsionen deutlich weniger als 0,5 Gew.-% eines oder mehrerer Emulgatoren enthalten bzw. sogar gänzlich emulgatorfrei sind.

**Stifte**

Weiterhin vorteilhaft im Sinne der Erfindung sind Zubereitungen in Form von Stiften. Technisch betrachtet sind die meisten Stiftformulierungen wasserfreie Fettmischungen aus festen oder halbfesten Wachsen und flüssigen Ölen, wobei hochgereinigte Paraffinöle und -wachse die Stiftgrundmasse darstellen.

Übliche Grundstoffe für stiftförmige Zubereitungen sind beispielsweise flüssige Öle (wie z. B. Paraffinöle, Ricinusöl, Isopropylmyristat, $C_{12-15}$ Alkylbenzoat), halbfeste Bestandteile (z. B. Vaseline, Lanolin), feste Bestandteile (z. B. Bienenwachs, Ceresin und mikrokristalline Wachse bzw. Ozokerit) und/oder hochschmelzende Wachse (z. B. Carnaubawachs, Candelillawachs). Auch wasserhaltige stiftförmige Zubereitungen sind an sich bekannt, wobei diese auch in Form von W/O-Emulsionen vorliegen können.

Die erfindungsgemäßen kosmetischen oder dermatologischen Lichtschutzformulierungen können wie üblich zusammengesetzt sein und dem kosmetischen oder dermatologischen Lichtschutz, ferner zur Behandlung, Pflege und Reinigung der Haut und/oder der Haare und als Schminkprodukt in der dekorativen Kosmetik dienen.

Entsprechend ihrem Aufbau können kosmetische oder topische dermatologische Zusammensetzungen im Sinne der vorliegenden Erfindung, beispielsweise verwendet werden als Hautschutzcreme, Reinigungsmilch, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Zur Anwendung werden die kosmetischen und dermatologischen Zubereitungen in der für Kosmetika üblichen Weise auf die Haut und/oder die Haare in ausreichender Menge aufgebracht.

Die kosmetischen und dermatologischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Vorteilhafte Komplexbildner im Sinne der vorliegenden Erfindung sind beispielsweise EDTA, [S,S]-Ethylendiamindisuccinat (EDDS), welches beispielsweise unter der Handelsbezeichnung Octaquest von der Fa. Octel erhältlich ist, Pentanatrium-Ethylendiamintetramethylenphosphonat, welches z. B. unter dem Handelsnamen Dequest 2046 von der Fa. Monsanto erhältlich ist und/oder Iminodibersteinsäure, welche u. a. von der Fa. Bayer AG unter den Handelsnamen Iminodisuccinat VP OC 370 (ca. 30% ige Lösung) und Baypure CX 100 fest erhältlich ist.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder ß-Alanin sowie 8-Hexadecen-1,16-dicarbonsäure (Dioic acid, CAS-Nummer 20701-68-2; vorläufige INCI-Bezeichnung Octadecendioic acid).

Erfindungsgemäße Rezepturen, welche z. B. bekannte Antifaltenwirkstoffe wie Flavonglycoside (insbesondere $\alpha$-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die Wasserphase der Zubereitungen gemäß der vorliegenden Erfindung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Bf. Goodrich], beispielsweise Carbopole der Typen 980, 981, 1382, 2984, 5984, ETD 2020, ETD 2050, Ultrez 10, jeweils einzeln oder in Kombination.

Die Zubereitungen gemäß der vorliegenden Erfindung können ferner vorteilhaft auch Selbstbräunungssubstanzen enthalten, wie beispielsweise Dihydroxyacteon und/oder Melaninderivate in Konzentrationen von 1 Gew.-% bis zu 8 Gew.-%, bezogen auf das Gesamtgewicht der Zubereitung.

Ferner vorteilhaft können die Zubereitungen gemäß der vorliegenden Erfindung auch Repellentien zum Schutz vor Mücken, Zecken und Spinnen und dergleichen enthalten. Vorteilhaft sind z. B. N,N-Diethyl-3-methylbenzamid (Handelsbezeichnung: Metadelphene, "DEET"), Dimethylphtalat (Handelsbezeichnung: Palatinol M, DMP) sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zur Prophylaxe und Behandlung kosmetischer oder dermatologischer Hautveränderungen, wie sie z. B. bei der Hautalterung auftreten, verwendet werden.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9).

Die Ölphase der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, $C_{12-13}$-Alkyllactat, Di-$C_{12-13}$-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an $C_{12-15}$-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Halistar AB*) und/oder Diethylhexylnaphthalat (*Hallbrite TQ oder Corapan TQ von H&R).*

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen

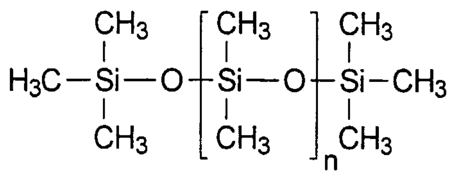

werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Die Zubereitungen gemäß der vorliegenden Erfindung können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:

(a) Siloxanelastomere, welche die Einheiten $R_2SiO$ und $RSiO_{1,5}$ und/oder $R_3SiO_{0,5}$ und/oder $SiO_2$ enthalten, wobei die einzelnen Reste R jeweils unabhängig voneinander Wasserstoff, $C_{1-24}$-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten $R_2SiO$ zu $RSiO_{1,5}$ aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
wobei die verwendeten Mengenateile so gewählt werden, daß die Menge des Wasserstoffes des Organopolysiloxans (1) oder der ungesättigten aliphatischen Gruppen des Organopolysiloxans (2)

- im Bereich von 1 bis 20 mol-% liegt, wenn das Organopolysiloxan nicht cyclisch ist und
- im Bereich von 1 bis 50 mol-% liegt, wenn das Organopolysiloxan cyclisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Ganz besonders bevorzugt ist es, wenn das Siloxanelastomer in Kombination mit unverzweigten bei Raumtemperatur flüssigen oder pastösen Silikonölen oder cyclischen Silikonölen oder deren Gemischen verwendet wird. Insbesondere vorteilhaft sind Organopolysiloxanelastomere mit der INCI-Bezeichnung Dimethicone / Polysilicone-11, ganz besonders die von der Grant Industries Inc. erhältlichen Gransil-Typen GCM, GCM-5, DMG-6, CSE Gel, PM-Gel, LTX, ININ Gel, AM-18 Gel und/oder DMCM-5.

Ganz außergewöhnlich bevorzugt ist es, wenn das Siloxanelastomer in Form eines Gels aus Siloxanelastomer und einer Lipidphase verwendet wird, wobei der Gehalt des Siloxanelastomers in dem Gel 1 bis 80 Gew.-%, bevorzugt 0,1 bis 60 Gew.-% beträgt, jeweils bezogen auf das Gesamtgewicht des Gels.

Es ist vorteilhaft im Sinne der vorliegenden Erfindung, die Gesamtmenge der Siloxanelastomere (Aktivgehalt) aus dem Bereich von 0,01 bis 10 Gew.-%, vorteilhaft von 0,1 bis 5 Gew.-%, jeweils bezogen auf das Gesamtgewicht der Formulierung, zu wählen.

Die erfindungsgemäßen kosmetischen und dermatologischen Zubereitungen können Farbstoffe und/oder Farbpigmente enthalten, insbesondere wenn sie in Form von dekorativen Kosmetika vorliegen. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. $Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus dem *Rowe Colour Index, 3. Auflage, Society of Dyers and Colourists, Bradford, England, 1971* zu wählen.

Sofern die erfindungsgemäßen Formulierungen in Form von Produkten vorliegen, welche im Gesicht angewendet werden, ist es günstig, als Farbstoff eine oder mehrere Substanzen aus der folgenden Gruppe zu wählen: 2,4-Dihydroxyazobenzol, 1-(2'-Chlor-4'-nitro-1'-phenylazo)-2-hydroxynaphthalin, Ceresrot, 2-(Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Ferner vorteilhaft sind öllösliche Naturfarbstoffe, wie z. B. Paprikaextrakte, β-Carotin oder Cochenille.

Vorteilhaft im Sinne der vorliegenden Erfindung sind ferner Formulierungen mit einem Gehalt an Perlglanzpigmenten. Bevorzugt sind insbesondere die im folgenden aufgelisteten Arten von Perlglanzpigmenten:

1. Natürliche Perlglanzpigmente, wie z. B.
   - "Fischsilber" (Guanin/Hypoxanthin-Mischkristalle aus Fischschuppen) und
   - "Perlmutt" (vermahlene Muschelschalen)
2. Monokristalline Perlglanzpigmente wie z. B. Bismuthoxychlorid (BiOCl)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteilhaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| Gruppe | Belegung / Schichtdicke | Farbe |
|---|---|---|
| Silberweiße Perlglanzpigmente | $TiO_2$: 40 - 60 nm | silber |
| Interferenzpigmente | $TiO_2$: 60 - 80 nm | gelb |
| | $TiO_2$: 80 - 100 nm | rot |
| | $TiO_2$: 100 - 140 nm | blau |
| | $TiO_2$: 120 - 160 nm | grün |
| Farbglanzpigmente | $Fe_2O_3$ | bronze |
| | $Fe_2O_3$ | kupfer |
| | $Fe_2O_3$ | rot |
| | $Fe_2O_3$ | rotviolett |
| | $Fe_2O_3$ | rotgrün |
| | $Fe_2O_3$ | schwarz |
| Kombinationspigmente | $TiO_2$ / $Fe_2O_3$ | Goldtöne |
| | $TiO_2$ / $Cr_2O_3$ | grün |
| | $TiO_2$ / Berliner Blau | tiefblau |
| | $TiO_2$ / Carmin | rot |

Besonders bevorzugt sind z. B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit $TiO_2$ und $Fe_2O_3$ beschichtete $SiO_2$-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Eisenperlglanzpigmente, welche ohne die Verwendung von Glimmer hergestellt werden. Solche Pigmente sind z. B. unter dem Handelsnamen Sicopearl Kupfer 1000 bei der Firma BASF erhältlich.

Besonders vorteilhaft sind ferner auch Effektpigmente, welche unter der Handelsbezeichnung Metasomes Standard / Glitter in verschiedenen Farben (yello, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an weiteren UV-Schutzsubstanzen enthalten. So werden z. B. in Tagescremes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise mindestens eine weitere UV-A-, UV-B- und/oder Breitbandfiltersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Die Zubereitungen gemäß der vorliegenden Erfindung können ferner vorteilhaft auch in Form von sogenannten ölfreien kosmetischen oder dermatologischen Emulsionen vorliegen, welche eine Wasserphase und mindestens eine bei Raumtemperatur flüssige UV-Filtersubstanz als weitere Phase enthalten und welche insbesondere vorteilhaft auch frei von weiteren Ölkomponenten sein können.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Ethylhexyl Salicylate) und Ester der Zimtsäure, vorzugsweise 4-Methoxyzimtsäure(2-ethylhexyl)ester (2-Ethylhexyl-4-methoxycinnamat, INCI: Ethylhexyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX bei Hoffmann La Roche erhältlich ist.

Bevorzugte anorganische Pigmente sind Metalloxide und/oder andere in Wasser schwerlösliche oder unlösliche Metallverbindungen, insbesondere Oxide des Titans ($TiO_2$), Zinks (ZnO), Eisens (z. B. $Fe_2O_3$), Zirkoniums ($ZrO_2$), Siliciums ($SiO_2$), Mangans (z. B. MnO), Aluminiums ($Al_2O_3$), Cers (z. B. $Ce_2O_3$), Mischoxide der entsprechenden Metalle sowie Abmischungen aus solchen Oxiden sowie das Sulfat des Bariums ($BaSO_4$).

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid ($Al_2O_3$), Aluminiumhydroxid $Al(OH)_3$, bzw. Aluminiumoxidhydrat (auch: Alumina, CAS-Nr.: 1333-84-2), Natriumhexametaphosphat $(NaPO_3)_6$, Natriummetaphosphat $(NaPO_3)_n$, Siliciumdioxid ($SiO_2$) (auch: Silica, CAS-Nr.: 7631-86-9), oder Eisenoxid ($Fe_2O_3$). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| Handelsname | Coating | Hersteller |
|---|---|---|
| Z- Cote HP1 | 2% Dimethicone | BASF |
| Z- Cote | / | BASF |
| ZnO NDM | 5% Dimethicone | H&R |
| MZ- 303S | 3% Methicone | Tayca Corporation |
| MZ- 505S | 5% Methicone | Tayca Corporation |

Geeignete Titandioxidpartikel und Vordispersionen von Titandioxidpartikeln sind unter folgenden Handelsbezeichnungen bei den aufgeführten Firmen erhältlich:

| Handelsname | Coating | Hersteller |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul $TiO_2$) | Octyltrimethylsilan | Degussa |
| Tioveil AQ 10PG | Alumina / Silica | Solaveil / Uniquema |
| Eusolex T-aqua | Wasser/Alumina/Natriummetaphosphat | Merck |

Weitere vorteilhafte Pigmente sind Latexpartikel. Erfindungsgemäß vorteilhafte Latexpartikel sind die in den folgenden Schriften beschriebenen: US 5,663,213 bzw. EP 0 761 201. Besonders vorteilhafte Latexpartikel sind solche, welche aus Wasser und Styrol/Acrylat-Copolymeren gebildet werden und z. B. unter der Handelsbezeichnung "Alliance SunSphere" bei der Fa. Rohm & Haas erhältlich sind.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Vorteilhafte weitere UV-Filtersubstanzen im Sinne der vorliegenden Erfindung sind sulfonierte, wasserlösliche UV-Filter, wie z. B.:

- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornyliden-methyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner Benzoxazol-Derivate, welche

sich durch die folgende Strukturformel auszeichnen,

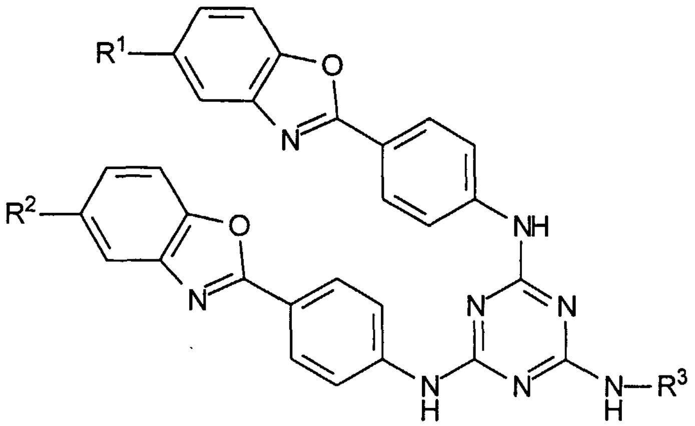

worin $R^1$, $R^2$ und $R^3$ unabhängig voneinander gewählt werden aus der Gruppe der verzweigten oder unverzweigten, gesättigten oder ungesättigten Alkylreste mit 1 bis 10 Kohlenstoffatomen. Es ist erfindungsgemäß besonders vorteilhaft, die Reste $R^1$ und $R^2$ gleich zu wählen, insbesondere aus der Gruppe der verzweigten Alkylreste mit 3 bis 5 Kohlenstoffatomen. Es ist ferner besonders vorteilhaft im Sinne der vorliegenden Erfindung, wenn $R^3$ einen unverzweigten oder verzweigten Alkylrest mit 8 Kohlenstoffatomen, insbesondere den 2-Ethylhexylrest darstellt.

Erfindungsgemäß besonders bevorzugtes Benzoxazol-Derivat ist das 2,4-bis-[5-1(dimethylpropyl)benzoxazol-2-yl-(4-phenyl)-imino]-6-(2-ethylhexyl)-imino-1,3,5-triazin mit der CAS Nr. 288254-16-0, welches sich durch die Strukturformel

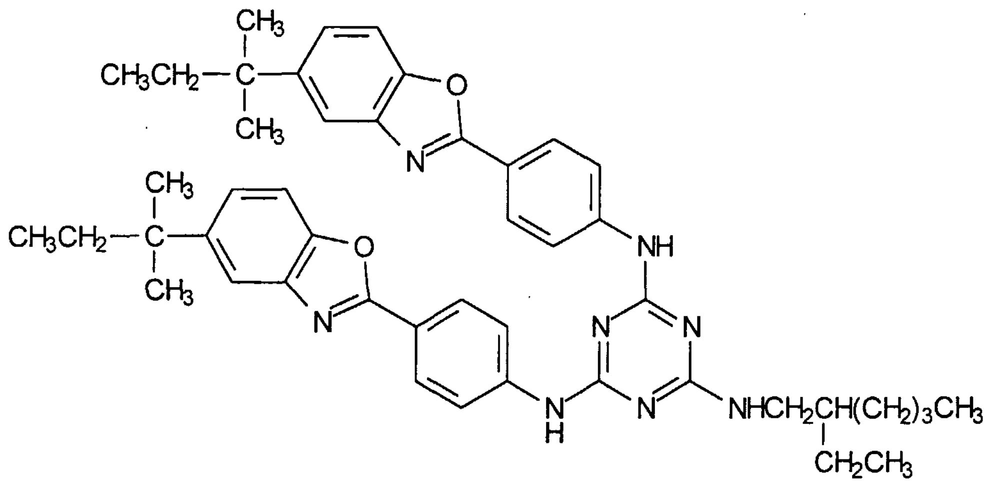

auszeichnet und bei 3V Sigma unter der Handelsbezeichnung Uvasorb® K2A erhältlich ist.

Das oder die Benzoxazol-Derivate liegen vorteilhaft in gelöster Form in den erfindungsgemäßen kosmetischen Zubereitungen vor. Es kann ggf. aber auch von Vorteil sein, wenn das oder die Benzoxazol-Derivate in pigmentärer, d. h. ungelöster Form - beispielsweise in Partikelgrößen von 10 nm bis zu 300 nm - vorliegen.

Vorteilhafte UV-Filtersubstanzen im Sinne der vorliegenden Erfindung sind ferner sogenannte Hydroxybenzophenone. Hydroxybenzophenone zeichnen sich durch die folgende Strukturformel aus:

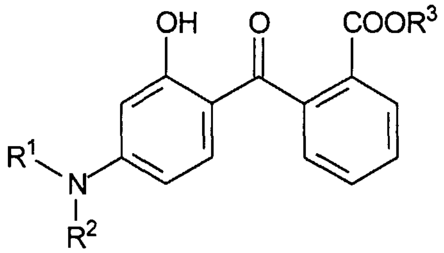

worin

- $R^1$ und $R^2$ unabhängig voneinander Wasserstoff, $C_1$-$C_{20}$-Alkyl, $C_3$-$C_{10}$-Cycloalkyl oder $C_3$-$C_{10}$-Cycloalkenyl bedeuten, wobei die Substituenten $R^1$ und $R^2$ gemeinsam mit dem Stickstoffatom, an das sie gebunden sind, einen 5- oder 6-Ring bilden können und
- $R^3$ einen $C_1$-$C_{20}$-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher sich durch folgende Struktur auszeichnet:

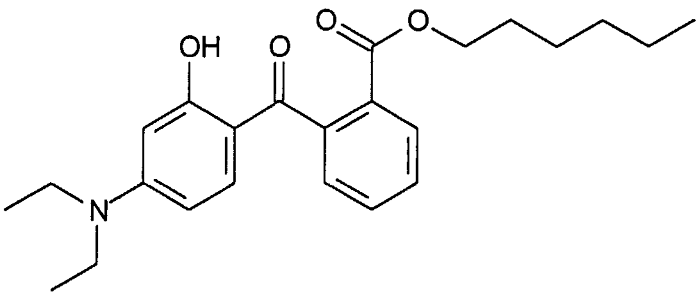

und unter dem Uvinul A Plus bei der Fa. BASF erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.

- 2,4-Bis-{[4-(2-Ethyl-hexyloxy)-2-hydroxy]-phenyl}-6-(4-methoxyphenyl)-1,3,5-triazin (INCI: Bis-Ethylhexyloxylphenol Methoxyphenyl Triazin), welches unter der Handelsbezeichnung Tinosorb® S bei der CIBA-Chemikalien GmbH erhältlich ist;
- Dioctylbutylamidotriazon (INCI: Diethylhexyl Butamido Triazone), welches unter der Handelsbezeichnung UVASORB HEB bei Sigma 3V erhältlich ist;
- 4,4',4"-(1,3,5-Triazin-2,4,6-triyltriimino)-tris-benzoesäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird;
- 2-[4,6-Bis(2,4-dimethylphenyl)-1,3,5-triazin-2-yl]-5-(octyloxy)phenol (CAS Nr.: 2725-22-6).

Ein vorteilhafter Breitbandfilter im Sinne der vorliegenden Erfindung ist auch das 2,2'-Me-thylen-bis-(6-(2H-benzotriazol-2-yl)-4-(1,1,3,3-tetramethylbutyl)-phenol) (INCI: Methylene Bis-Benztriazolyl Tetramethylbutylphenol), welches z.B. unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die weiteren UV-Filtersubstanzen können öllöslich oder wasserlöslich sein. Vorteilhafte öllösliche Filtersubstanzen sind z. B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;

- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter.

Vorteilhafte wasserlösliche Filtersubstanzen sind z. B.:

Sulfonsäure-Derivate des 3-Benzylidencamphers, wie z. B. 4-(2-Oxo-3-bornylidenme-thyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 T erhältlich ist.

Besonders vorteilhafte Zubereitungen im Sinne der vorliegenden Erfindung, die sich durch einen hohen bzw. sehr hohen UV-A-Schutz auszeichnen, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan] und/oder das 2,4-Bis-{[4-(2-Ethyl-hexyloxy)-2-hydroxy]-phenyl}-6-(4-methoxyphenyl)-1,3,5-triazin und/oder Phenylen-1,4-bis-(2-benzimidazyl)-3,3'-5,5'-tetrasulfonsäure-bis-natriumsalz jeweils einzeln oder in beliebigen Kombinationen miteinander.

Die Liste der genannten UV-Filter, die im Sinne der vorliegenden Erfindung eingesetzt werden können, soll selbstverständlich nicht limitierend sein.

Vorteilhaft enthalten die Zubereitungen gemäß der vorliegenden Erfindung die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Die Zubereitungen im Sinne der vorliegenden Erfindung können ferner vorteilhaft weitere Substanzen enthalten, die die Wasserfestigkeit der Produkte erhöhen.

Vorteilhaft sind beispielsweise wasserlösliche oder in Wasser dispergierbare Polyoxyethylen-Polyoxypropylen-Blockpolymere (CTFA-Bezeichnung: Polaxamere, CAS-Nr. 9003-11-6) mit folgender Struktur:

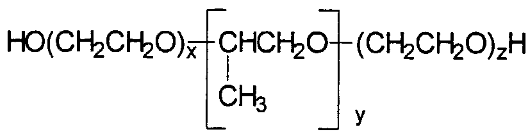

wobei x, y und z ganze Zahlen aus dem Bereich von 2 bis 130, insbesondere von 15 bis 100 darstellen und x und z gleich sind, aber unabhängig von y gewählt werden.

Unter diesen sind insbesondere Polaxamer 188 [mit x = 75, y = 30 und z = 75], welches unter der Handelsbezeichnung Lutrol F 68 (alt: Pluronic F 68) von der Firma BASF zu beziehen ist, das Polyxamer 185 [mit x = 19, y = 30 und z = 19] (Lubrajel WA von ISP), das Polyxamer 235 [mit x = 27, y = 39 und z = 27] (Pluronic F 85 von BASF) und/oder das Polyxamer 238 [mit x = 97, y = 39 und z = 97] (Pluronic F 88 von BASF) vorteilhaft zu verwenden.

Weitere vorteilhafte Substanzen, welche zur Steigerung der Wasserfestigkeit beitragen können, aber in die Ölphase der Zubereitungen gemäß der vorliegenden Erfindung eingearbeitet werden, sind bestimmte Wachskomponenten, wie acetyliertes Glykolstearat mit Tristearin (z. B. Unitwix von ISP mit der INCI: Acetylated Glycol Stearat and Tristearin), $C_{18-36}$ Fettsäuretriglycerid (z. B: Syncrowax HGLC von der Fa. Crode GmbH mit der INCI: C18-36 Acid Triglyceride) sowie die unter den Handelsbezeichnungen "Performa V 1608" (INCI: C30-38 Olefin/Isopropyl Maleate/MA Copolymer) und "Perfroma V 825" (synethisches Wachs) von New Phase Technologies erhältlichen Substanzen.

Es ist besonders vorteilhaft im Sinne der vorliegenden Erfindung die erfindungsgemäß verwendeten Polymere mit einer oder mehreren der genannten Substanzen zu kombinieren, um die Wasserfestigkeit der Zubereitungen noch weiter zu verbessern.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

**Beispiele:**

In den folgenden Beispielen bedeutet:

- UVASorb® K2A = 2,4-bis-[5-1(dimethylpropyl)benzoxazol-2-yl-(4-phenyl)-imino]-6-(2-ethylhexyl)-imino-1,3,5-triazin [CAS Nr. 288254-16-0]
- Uvinul® A Plus = 2-(4'-Diethylamino-2'-hydoxybenzoyl)-benzoesäurehexylester (auch: Aminobenzophenon)

**1. O/W Sonnenschutz Emulsionen**

| | 1 | 2 | 3 | 4 | 5 | 6 | 7 |
|---|---|---|---|---|---|---|---|
| Glycerinmonostearat SE | 0,50 | 1,00 | 3,00 | | | 1,50 | |
| Glyceryl Stearat Citrat | 2,00 | | | 1,00 | 2,00 | | 2,50 |
| Stearinsäure | | 3,00 | 0,75 | 2,00 | | | |
| PEG-100 Stearat | | | 1,50 | | | 2,00 | |
| Lauryl Methicon Copolyol | | | | 0,75 | | 0,50 | |
| Cetyl Phosphat | | | 0,75 | | | | |
| Stearyl Alkohol | | | 3,00 | | | 2,00 | 0,50 |
| Cetyl Alkohol | 2,50 | | | | 0,50 | | 2,00 |
| UVASorb® K2A | | | 3,00 | | | | |
| Uvinul® A Plus | 3,00 | 2,50 | | | | | |
| Bis-Ethylhexyloxyphenol Methoxyphenyl Triazin | | 1,00 | | 2,00 | | 1,00 | 0,50 |
| Disodium Phenyl Dibenzimidazol Tetrasulfonat | | | | | 0,75 | 2,00 | |
| Ethylhexyl Triazon | 2,00 | | | 2,00 | | 2,00 | |
| Diethylhexyl Butamido Triazon | | 2,00 | | | | | |
| Ethylhexyl Methoxycinnamat | | 3,50 | | 10,00 | | | |
| Octocrylen | | | | 5,00 | 9,00 | 7,50 | 2,50 |
| Methylen Bis-Benztriazolyl Tetramethylbutylphenol | | | 2,00 | | 3,00 | | |
| Ethylhexylsalicylat | | | 3,00 | | | | 5,00 |
| Drometrizol Trisiloxan | | | 0,50 | | | 1,00 | |
| Titandioxid T 805 | | 1,50 | | | 1,00 | 0,50 | |
| Titandioxid MT-100Z | 1,00 | | | 3,00 | 1,00 | | |
| $C_{12-15}$ Alkylbenzoat | | 2,50 | | | | 7,00 | 5,00 |
| Dicaprylyl Ether | | | 3,50 | | 2,00 | | |
| Butylenglycol Dicaprylat/Dicaprat | 5,00 | | | 5,00 | 3,00 | | |
| Cetearyl Isononanoate | | 4,00 | | | | 2,00 | 2,00 |
| Dimethicon | | 0,50 | 1,00 | | 2,00 | | |
| Cyclomethicon | 2,00 | | | 4,50 | | | 0,50 |
| Dimethicon / Vinyl Dimethicon Crosspolymer | | 4,00 | | | | | 0,50 |
| Polaxamer 188 | 1,00 | | | | | | |
| PEG-45 Dodecyl Gycol Copolymer | 0,75 | 0,50 | 0,50 | 0,75 | 1,50 | | 0,25 |
| PEG-22 Dodecyl Gycol Copolymer | | | | | | 0,75 | |

(fortgesetzt)

| | 1 | 2 | 3 | 4 | 5 | 6 | 7 |
|---|---|---|---|---|---|---|---|
| Acetyliertes Gylcol Stearate + Tristearin | | | | | | | 3,00 |
| PVP Hexadecen Copolymer | 0,50 | | | 0,50 | 1,00 | | 1,00 |
| Glycerin | 3,00 | 7,50 | | 7,50 | 5,00 | | 2,50 |
| Xanthan Gummi | 0,15 | | 0,05 | | | | 0,30 |
| Vitamin E Acetat | 0,50 | | 0,25 | 0,50 | 0,75 | | 1,00 |
| Fucogel® 1000 | | | 1,50 | | | 5,00 | |
| DMDM Hydantoin | | 0,60 | 0,40 | 0,20 | | | |
| Methylparaben | 0,15 | | 0,25 | | 0,50 | | |
| Phenoxyethanol | 1,00 | 0,40 | | 0,40 | 0,50 | | 0,60 |
| EDTA | | 0,20 | 0,35 | 0,50 | 0,02 | | 0,03 |
| Ethanol | | 2,00 | 1,50 | | | 5,00 | |
| Insekt Repellent 3535 | | | | | 5,00 | | |
| Parfüm | 0,20 | 0,20 | | | | 0,30 | 0,40 |
| Wasser | ad 100 | ad 100 | ad 100 | ad 100 | ad 100 | ad 100 | ad 100 |

**2. schaumförmige O/W- Emulsionen:**

| | Emulsion 1 | | Emulsion 2 | |
|---|---|---|---|---|
| | GEW.-% | Vol.-% | GEW.-% | Vol.-% |
| Stearinsäure | 5,00 | | 1,00 | |
| Cetylalkohol | 5,50 | | | |
| Cetylstearylalkohol | | | 2,00 | |
| PEG-40 Stearat | 8,50 | | | |
| PEG-20-Stearat | | | 1,00 | |
| Caprylsäure/Caprinsäuretriglyceride | 4,00 | | 2,00 | |
| $C_{12-15}$ Alkylbenzoat | 10,00 | | 15,50 | |
| Cyclomethicon | 4,00 | | | |
| Dimethicon | | | 0,50 | |
| Octylisostearat | | | 5,00 | |
| Myristyl Myristat | | | 2,00 | |
| Ceresin | 1,50 | | | |
| Glycerin | | | 3,00 | |
| Butylmethoxydibenzoylmethan | 1,00 | | | |
| Bis-Ethylhexyloxyphenol Methoxyphenyl Triazin | 1,50 | | 2,00 | |
| Disodium Phenyl Dibenzimidazol Tetrasulfonat | 1,50 | | | |
| Ethylhexylmethoxycinnamat | 5,00 | | 4,00 | |
| Ethylhexyltriazon | | | 3,00 | |
| Octocrylene | 5,00 | | | |

(fortgesetzt)

| | Emulsion 1 | | Emulsion 2 | |
|---|---|---|---|---|
| | GEW.-% | Vol.-% | GEW.-% | Vol.-% |
| Titandioxid MT 100 TV | | | 2,00 | |
| PEG-45 Dodecyl Gycol Copolymer | 0,50 | | 1,00 | |
| Polaxamer 188 | | | 1,50 | |
| BHT | | | 0,02 | |
| $Na_2H_ZEDTA$ | 0,50 | | 0,10 | |
| Parfüm, Konservierungsmittel, | Q.S. | | Q.S. | |
| Farbstoffe, usw. | Q.S. | | Q.S. | |
| Kaliumhydroxid | Q.S | | Q.S | |
| Wasser | AD 100,00 | | AD 100,00 | |
| | pH-Wert eingestellt auf 6,5-7,5 | | pH-Wert eingestellt auf 5,0-6,0 | |
| **Emulsion 1** | | 70 | | |
| **Emulsion 2** | | | | 35 |
| Gas (Stickstoff) | | 30 | | |
| Gas (Helium) | | | | 65 |

Vereinigung der auf 78 °C aufgeheizten Fett-/Lichtschutzfilterphase mit der auf 75 °C aufgeheizten Wasser-/Lichtschutzfilterphase. Homogenisierung mittels einer Zahnkranzdispergiermaschine (Rotor-Stator-Prinzip) bei 65 °C. 45 min Rühren im Becomix unter Begasung mit Helium bei 1 bar unter Kühlung auf 30 °C. Zugabe der Additive bei 30 °C (Parfüm). Homogenisierung mittels einer Zahnkranzdispergiermaschine (Rotor-Stator-Prinzip) bei 23 °C.

**3. PIT-Emulsionen (zur Verwendung als Tränkungslösung, Spray oder Aerosol)**

| | 1 | 2 | 3 | 4 | 5 | 6 | 7 | 8 |
|---|---|---|---|---|---|---|---|---|
| Glycerinmonostearat SE | 0,50 | 2,00 | 3,00 | 5,00 | | | 0,50 | 4,00 |
| Glyceryl Isostearat | | | | | 3,50 | 4,00 | 2,00 | |
| Isoceteth-20 | | 0,50 | 0,50 | | 2,00 | | | |
| Ceteareth-12 | | 5,00 | | 1,00 | | | | 3,50 |
| Ceteareth-20 | | | | 2,00 | | 2,50 | 3,00 | |
| Cetyl Alkohol | 2,50 | 1,00 | | 1,50 | 1,50 | 0,50 | 1,50 | |
| Cetyl Palmitat | | | | 0,50 | | 1,00 | | |
| Polyglyceryl-2 Dipolyhydroxystearat | | | 0,75 | | 0,25 | | | |
| UVASorb® K2A | | | | | | 3,00 | | |
| Uvinul® A Plus | 2,50 | | | | | | | |
| Dinatrium Phenyl Dibenzimidazol Tetrasulfonat | | 2,00 | | | | | | 2,00 |
| Phenylbenzmidazol Sulfonsäure | 1,00 | | | 2,00 | 3,00 | | | 2,50 |
| Bis-Ethyhexyloxyphenol Methoxyphenyl Triazin | | | 1,50 | 2,00 | | 2,00 | 1,00 | |

(fortgesetzt)

| | 1 | 2 | 3 | 4 | 5 | 6 | 7 | 8 |
|---|---|---|---|---|---|---|---|---|
| Butyl Methoxydibenzoylmethan | | | 1,00 | | 2,00 | | 0,75 | |
| Ethylhexyl Methoxycinnamat | 8,00 | | | 4,50 | | 10,00 | | |
| Diethylhexyl Butamido Triazon | | | | | 1,00 | | | 1,50 |
| Ethylhexyl Triazon | 2,00 | | 2,00 | 1,00 | | 2,00 | | 3,00 |
| Octocrylen | | | 5,00 | | 10,00 | | | 7,50 |
| $C_{12-15}$ Alkylbenzoat | 3,50 | | | 4,00 | | | | 3,50 |
| Cocoglyceride | | 3,00 | | | | | | |
| Dicaprylyl Ether | 4,00 | | 2,00 | | | | | |
| Butylenglycol Dicaprylat/Dicaprat | | 4,00 | | 2,50 | | | | |
| Dicaprylyl Carbonat | | | 5,00 | | | | | 6,00 |
| PEG-45 Dodecyl Gylcol Copolymer | | 0,75 | | 0,50 | 0,75 | 1,00 | 1,25 | 0,50 |
| Methoxy PEG-22 Dodecyl Glycol Copolymer | 1,00 | | 1,50 | | | | | |
| Polaxamer 188 | | | | | | | | 1,00 |
| Acetyliertes Gylcol Stearate + Tristearin | 0,50 | | | 1,00 | | | 3,00 | |
| PVP Hexadecen Copolymer | | | 0,50 | | | 0,50 | | 0,50 |
| Glycerin | 10,0 | 5,00 | | 7,50 | | 10,00 | | |
| Vitamin E Acetat | 1,00 | | | 0,75 | 0,50 | | 1,00 | |
| Iodopropyl Butylcarbamat | 0,12 | - | | | 0,20 | | | |
| DMDM Hydantoin | | | | 0,10 | | 0,05 | | |
| Methylparaben | | 0,50 | | | 0,45 | | | |
| Phenoxyethanol | 0,50 | 0,40 | | 1,00 | | | | 1,00 |
| Ethylhexyloxyglycerin | | 0,30 | | | 1,00 | 0,35 | | |
| Ethanol | | | 5,00 | | | | 7,50 | 4,00 |
| Farbstoffe, wasserlöslich | | | | | | 0,01 | | 0,01 |
| Trisodium EDTA | | | 0,14 | | | 0,20 | | 0,50 |
| Parfüm | 0,20 | | 0,20 | 0,20 | 0,45 | | | 0,20 |
| Wasser | ad. 100 | ad. 100 | ad. 100 | ad. 100 | ad. 100 | ad. 100 | ad. 100 | ad. 100 |

**4. Dünnflüssige bis sprühbare W/O-Emulsionen (zur Verwendung als Tränkungslösung, Spray oder Aerosol)**

| | 1 | 2 | 3 | 4 | 5 |
|---|---|---|---|---|---|
| Cetyl Dimethicon Copolyol | 4,00 | | | 2,50 | 3,00 |
| Polyglyceryl-2 Dipolyhydroxystearat | | | 3,00 | | 1,00 |
| Isostearyl Diglyceryl Succinat | | | 0,75 | | 0,30 |

(fortgesetzt)

| | 1 | 2 | 3 | 4 | 5 |
|---|---|---|---|---|---|
| Lauryl Methicon Copolyol | | | | 2,00 | |
| Polysorbat-65 | | | 2,00 | | 1,50 |
| PEG-100 Stearat | | | | 1,20 | 0,70 |
| Dimethicon | | 4,00 | | | 2,00 |
| Cyclomethicon | 12,00 | 20,00 | | 30,00 | 15,00 |
| Uvinul® A Plus | 3,50 | | | | 0,25 |
| Butylmethoxydibenzoylmethan | | 2,00 | | | |
| Bis-Ethylhexyloxyphenol Methoxyphenyl Triazin | 1,00 | | 2,00 | 0,50 | |
| Dinatrium Phenyl Dibenzimidazol Tetrasulfonat | | 1,50 | | | 2,00 |
| Drometrizol Trisiloxan | | | | 1,00 | |
| Ethylhexyl Methoxycinnamat | 3,00 | 4,00 | | | 10,00 |
| Ethylheyl Salicylat | | | 5,00 | | 3,50 |
| Octocrylen | | 5,00 | | 4,00 | |
| Diethylhexyl Butamido Triazon | | 1,00 | | | 6,50 |
| Ethylhexyl Triazon | 3,00 | | | | 4,00 |
| Titandioxid MT-100 Z | | 0,50 | 1,00 | 1,50 | 0,50 |
| Zinkoxid Z-Cote | 2,00 | | | | 4,00 |
| Dicaprylyl Carbonat | 5,00 | | 15,00 | | 4,00 |
| Dihexyl Carbonat | | 10,00 | | | |
| $C_{12-15}$ Alkylbenzoat | 7,00 | | 10,00 | | |
| Mineral Öl | 10,00 | | | | 6,00 |
| Cocoglyceride | | 2,00 | | 5,00 | |
| Acetyliertes Gylcol Stearate + Tristearin | 0,50 | | | 3,00 | |
| PEG-45 Dodecyl Gycol Copolymer | 0,75 | | 1,00 | 1,25 | 0,50 |
| Methoxy PEG-22 Dodecyl Glycol Copolymer | | 0,75 | | | |
| PVP Hexadecen Copolymer | | 0,75 | | | 0,40 |
| Glycerin | | | | 5,00 | 7,00 |
| $\alpha$-Glucosylrutin | | | | | 0,15 |
| EDTA | | 0,15 | 0,03 | | 0,15 |
| Glycin Soja | 0,75 | | | 1,50 | |
| Magnesiumsulfat | 0,75 | 1,00 . | | 0,45 | 1,00 |
| DMDM Hydantoin | | 0,05 | | | 0,10 |
| Phenoxyethanol | 1,00 | 0,75 | 0,50 | | 1,00 |
| Ethanol | 2,00 | | | 5,00 | 1,00 |
| Farbstoff, öllöslich | 0,02 | | | | |
| Parfüm | 0,30 | 0,45 | 0,35 | | 0,15 |

(fortgesetzt)

| | 1 | 2 | 3 | 4 | 5 |
|---|---|---|---|---|---|
| Wassser | ad. 100 | ad. 100 | ad. 100 | ad. 100 | ad. 100 |

**5. W/O Sonnenschutz Emulsionen (Cremes & Lotions)**

| | 1 | 2 | 3 | 4 | 5 |
|---|---|---|---|---|---|
| Cetyldimethicon Copolyol | | | | 4,00 | |
| Polyglyceryl-2-dipolyhydroxystearat | 5,00 | 4,50 | | | 4,50 |
| PEG-30-dipolyhydroxystearat | | | 5,00 | 2,00 | |
| PEG-45 Dodecyl Gylcol Colpolymer | | | | | 1,00 |
| UVASorb® K2A | | | | 2,75 | |
| Uvinul ® A Plus | | 3,25 | | | |
| Bis-Ethylhexyloxyphenol Methoxyphenyl Triazin | 1,75 | | 1,50 | 2,00 | 3,00 |
| Phenylbenzmidazol Sulfonsäure | | 4,00 | 2,00 | | 0,50 |
| Ethylhexyl Methoxycinnamat | | 8,00 | | 5,00 | 4,00 |
| Diethylhexyl Butamido Triazon | 3,00 | 1,00 | | | 3,00 |
| Ethylhexyl Triazon | | | 3,00 | 4,00 | |
| Octocrylen | 7,00 | | 8,00 | | 2,50 |
| Drometrizol Trisiloxan | | | 3,00 | | |
| Titandioxid Uvinul® T 805 | 2,00 | 1,00 | | | |
| Titandioxid MT-100 TV | | | 3,00 | | 2,00 |
| Zinkoxid Z-Cote® HP1 | 2,50 | | 6,00 | | |
| Mineralöl | | | 10,0 | | 8,00 |
| Cocoglyceride | 4,00 | 6,50 | | | |
| $C_{12-15}$ Alkylbenzoat | | | | 9,00 | |
| Dicaprylyl Ether | 10,00 | | | | 7,00 |
| Butylenglycol Dicaprylat/Dicaprat | | | 2,00 | 8,00 | 4,00 |
| Cyclomethicon | 2,00 | | | | 2,00 |
| PVP Eicosene Copolymer | 0,50 | | | 1,50 | 1,00 |
| Polaxamer 188 | 0,50 | | | 1,00 | |
| Acetyliertes Gylcol Stearate + Tristearin | | | 1,00 | | |
| PEG-45 Dodecyl Gycol Copolymer | 0,50 | 1,00 | 1,25 | | 0,50 |
| Methoxy PEG-22 Dodecyl Glycol Copolymer | | | | 1,00 | |
| Trinatrium EDTA | 1,00 | | | 0,35 | |
| Ethylhexyloxyglycerin | | 0,30 | 1,00 | | 0,50 |
| Glycerin | 3,00 | 7,50 | | 7,50 | 2,50 |
| Butylen Glykol | | | 10,00 | | 6,50 |
| Glycin Soja | | 1,00 | 1,50 | | |
| $MgSO_4$ | 1,00 | 0,50 | | 0,50 | |

(fortgesetzt)

| | 1 | 2 | 3 | 4 | 5 |
|---|---|---|---|---|---|
| Vitamin E | 0,50 | | 0,25 | | 1,00 |
| DMDM Hydantoin | | 0,60 | | 0,20 | |
| Methylparaben | 0,50 | | | 0,15 | |
| Phenoxyethanol | 0,50 | 0,40 | | 1,00 | 0,60 |
| Ethanol | 3,00 | | 4,50 | | 1,00 |
| Parfüm | 0,20 | | 0,20 | | 0,20 |
| Wasser | ad 100 | ad 100 | ad 100 | ad 100 | ad 100 |

**6. Hydrodispersionen (zur Verwendung als Lotion, Tränkungslösung oder Spray)**

| | 1 | 2 | 3 | 4 | 5 |
|---|---|---|---|---|---|
| PEG-40 Stearat | | 1,25 | | | |
| Cetyl Alkohol | | | | 2,00 | |
| Sodium Carbomer | | 0,20 | | 0,30 | |
| Acrylates/C 10-30 Alkyl Acrylate Crosspolymer | | | 0,40 | 0,10 | 0,10 |
| Xanthan Gummi | 0,50 | 0,30 | 0,15 | | 0,50 |
| Dimethicon / Vinyl Dimethicon Crosspolymer | | | 5,00 | | 3,00 |
| Uvinul® A Plus | | | | 2,00 | |
| Bis-Ethylhexyloxyphenol Methoxypheny Triazin | 3,00 | 2,00 | 0,25 | | 1,75 |
| Terephthaliden Dicampher Sulfonsäure | | 0,20 | | 0,75 | |
| Dinatrium Phenyl Dibenzimidazol Tetrasulfonat | 0,75 | | 3,50 | | |
| Ethylhexyl Methoxycinnamat | | | | 5,00 | 8,00 |
| Methylen Bis-Benzotriazolyl Tetramethylbutylphenol | | 1,00 | | | 2,00 |
| Diethylhexyl Butamido Triazon | | | 2,00 | | |
| Ethylhexyl Triazon | 4,00 | | | 4,00 | |
| Octocrylen | | 4,00 | 10,00 | | 2,50 |
| Titandioxid T 805 | 0,50 | | 2,00 | 3,00 | 1,00 |
| $C_{12-15}$ Alkylbenzoat | 2,00 | 2,50 | | | |
| Butylenglycol Dicaprylat/Dicaprat | 4,00 | | | 6,00 | |
| Dicaprylyl Carbonat | | 3,00 | | | |
| Cyclomethicon | | | 7,50 | | |
| Lanolin | | | | 0,35 | |
| PVP Hexadecen Copolymer | 0,50 | | | 0,50 | 1,00 |
| Polaxamer 188 | | | | | 1,00 |
| PEG-45 Dodecyl Gycol Copolymer | 1,00 | 0,75 | 0,50 | 1,00 | 1,25 |
| Acetyliertes Gylcol Stearate + Tristearin | | 2,00 | | 1,00 | |
| C18-36 Triglycerid Fettsäuren | | | 1,00 | | |
| Glycerin | 3,00 | 7,50 | | 7,50 | 2,50 |

(fortgesetzt)

| | 1 | 2 | 3 | 4 | 5 |
|---|---|---|---|---|---|
| Glycin Soja | | 1,50 | 1,00 | | |
| Vitamin E Acetat | 0,50 | 0,20 | 0,25 | 0,75 | 1,00 |
| α - Glycosil Rutin | | 0,30 | | 0,25 | |
| Trinatrium EDTA | | 0,30 | 0,10 | 0,20 | |
| Konkaben LMB ® | 0,20 | | | | 0,15 |
| Methylparaben | 0,50 | | | 0,15 | |
| Phenoxyethanol | 0,50 | | | 1,00 | 0,60 |
| Ethanol | 3,00 | 7,00 | 3,50 | | 1,00 |
| Parfüm | 0,20 | | 0,20 | 0,40 | 0,20 |
| Farbstoffe, wasserlöslich | | | 0,02 | | |
| Wasser | ad 100 | ad 100 | ad 100 | ad 100 | ad 100 |

**7. Feststoffstablisisierte Emulsionen**

| | 1 | 2 | 3 | 4 | 5 |
|---|---|---|---|---|---|
| Mineralöl | | | 16,00 | 16,00 | |
| Octyldodecanol | 9,00 | 9,00 | 5,00 | | |
| Caprylic/Capric Triglycerid | 9,00 | 9,00 | 6,00 | | |
| $C_{12-15}$ Alkylbenzoat | | | | 5,00 | 8,00 |
| Butylene Glycol Dicaprylate/Dicaprate | | | | | 8,00 |
| Dicaprylyl Ether | 9,00 | | | 4,00 | |
| Dicaprylyl Carbonate | | 9,00 | | | |
| Hydroxyoctacosanyl Hydroxystearate | 2,00 | 2,00 | 2,00 | 2,00 | 1,50 |
| Disteardimonium Hectorit | 1,00 | 0,750 | 0,50 | 0,50 | 0,25 |
| Cera Microcristallina + Paraffinum Liquidum | | | 2,50 | | 5,00 |
| Hydroxypropyl Methylcellulose | 0,15 | | | | 0,05 |
| Dimethicon | | | 4,50 | | |
| UVASorb® K2A | | 3,00 | | | |
| Uvinul® A Plus | | | | 3,15 | |
| Bis-Ethylhexyloxyphenol Methoxypheny Triazin | 1,75 | 2,00 | 0,75 | | 1,00 |
| Phenylbenzmidazol Sulfonsäure | 2,00 | 0,50 | | | |
| Ethylhexylmethoxycinnamat | 6,00 | | | | 3,0 |
| Octocrylen | 3,50 | | 7,50 | | |
| Ethylhexyl Salicylat | | 3,50 | | | 4,00 |
| Diethylhexyl Butamido Triazon | | | | | 4,0 |
| Titandioxid Eusolex ® T-2000 | | 2,00 | 4,00 | 2,00 | 4,00 |
| PEG-45 Dodecyl Gycol Copolymer | 2,00 | 0,75 | 0,50 | 0,75 | 1,25 |
| Acetyliertes Gylcol Stearate + Tristearin | 2,00 | | | 1,50 | |

(fortgesetzt)

| | 1 | 2 | 3 | 4 | 5 |
|---|---|---|---|---|---|
| Silica Dimethyl Silylat | | | 1,00 | | |
| Bornitrid | 4,00 | | | 3,00 | |
| Tapioca Stärke | | | | 1,00 | |
| Natrium Chlorid | 1,00 | 1,00 | 1,00 | 1,00 | |
| Glycerin | 5,0 | 10,0 | | 6,00 | 10,0 |
| Trinatrium EDTA | | 1,00 | | 1,00 | |
| Methylparaben | 0,21 | | | | 0,20 |
| Propylparaben | 0,07 | | | | |
| Phenoxyethanol | 0,50 | | 0,40 | 0,40 | 0,50 |
| Hexamidin Diisethionat | | | | | 0,08 |
| Diazolidinyl Harnstoff | | | 0,28 | 0,28 | |
| Alcohol | | 5,00 | | 2,50 | |
| Parfüm | 0,45 | 0,20 | | | 0,45 |
| Wasser | ad 100 | ad 100 | ad 100 | ad 100 | ad 100 |

**8. Öle und Ölgele**

| | 1 | 2 | 3 | 4 | 5 |
|---|---|---|---|---|---|
| Octyldodecanol | 9,00 | 9,00 | | | |
| Caprylic/Capric Triglycerid | 9,00 | | 6,00 | | |
| C12-15- Alkyl Benzoate | | | | 5,00 | 8,00 |
| Butylene Glycol Dicaprylate/Dicaprate | | 9,00 | | | 8,00 |
| Dicaprylyl Ether | 9,00 | | | 4,00 | |
| Dicaprylyl Carbonate | | 7,00 | | | |
| Ethyl Galaktomannan (N-Hance® AG 200) | 4,50 | | | | 4,00 |
| C20-40 Fettsäuren + Polyethylene (Performacid®350) | | | | 3,60 | |
| PEG-45 Dodecyl Gycol Copolymer | 0,75 | 1,00 | 1,25 | 0,50 | 0,50 |
| Acetyliertes Gylcol Stearate + Tristearin | 1,00 | | 2,00 | 1,00 | |
| Hydroxyoctacosanyl Hydroxystearate | 2,00 | | | | |
| Disteardimonium Hectorit | 1,00 | | | | 1,00 |
| Cetyl Dimethicon | 0,50 | | 4,50 | | |
| Cyclomethicone | | | 15,00 | | 5,00 |
| Butylmethoxidibenzoylmethan | 2,00 | | | | |
| Uvinul® A Plus | | 1,50 | | | 3,00 |
| Bis-Ethylhexyloxyphenol Methoxypheny Triazin | | | 0,75 | 2,00 | |
| Ethylhexylmethoxycinnamat | 6,00 | | | 10,00 | 3,0 |
| Octocrylen | 3,50 | | 7,50 | 10,00 | |
| Ethylhexyl Salicylat | | 3,50 | | | 4,00 |

(fortgesetzt)

| | 1 | 2 | 3 | 4 | 5 |
|---|---|---|---|---|---|
| Ethylhexyl Triazon | | | 2,00 | | |
| Diethylhexyl Butamido Triazon | | 0,50 | | 3,00 | 4,0 |
| Phenoxyethanol | 0,50 | | | | |
| Parfüm | 0,45 | 0,20 | | 0,45 | 0,45 |
| Farbstoff, öllöslich | | 0,02 | | | 0,025 |
| Mineralöl | ad. 100 | ad. 100 | ad. 100 | | |
| Reisöl | | | | ad. 100 | ad. 100 |

**9. Sonnenschutz Stifte-(für Lippen und/oder Gesicht)**

| | 1 | 2 | 3 | 4 |
|---|---|---|---|---|
| Caprylic/Capric Triglycerid | 12,00 | 10 | 6 | |
| Octyldodecanol | 7,00 | 14 | 8 | 3 |
| Butylene Glycol Dicaprylat/Dicaprat | | | | 12 |
| Pentaerythrityl Tetraisostearat | 10,00 | 6 | 8 | 7 |
| Polyglyceryl-3 Diisostearat | 2,50 | | | |
| Bis-Diglyceryl Polyacyladipate-2 | 9,00 | 8,00 | 10,00 | 8,00 |
| Cetearyl Alcohol | - 8,00 | 11,00 | 9,00 | 7,00 |
| Myristyl Myristate | 3,50 | 3,00 | 4,00 | 3,00 |
| Beeswax | 5,00 | 5,00 | 6,00 | 6,00 |
| Cera Carnauba | 1,50 | 2,00 | 2,00 | 1,50 |
| Cera Alba | 0,50 | 0,50 | | 0,50 |
| $C_{16-40}$ Alkylstearat | | 2,50 | 1,50 | 1,50 |
| PEG-45 Dodecyl Gycol Copolymer | 0,75 | 1,25 | 2,00 | 1,00 |
| Acetyliertes Gylcol Stearate + Tristearin | | | 3,00 | |
| UVASorb® K2A | 0,45 | | | |
| Uvinul® A Plus | | | | 1,00 |
| Bis-Ethylhexyloxyphenol Methoxyphenyl Triazin | 2,50 | | 0,25 | |
| Ethylhexyl Triazon | 2,00 | | | |
| Diethylhexyl Butamido Triazon | | | | 3,00 |
| Drometrizol Trisiloxan | 1,00 | | | |
| Z-Cote® HP1 | | 7,50 | 2,00 | 4,50 |
| MT-100 TV | | 4,00 | 2,50 | |
| Titandioxid T 805 | | 3,60 | | 5,00 |
| Ethylhexyl Methoxycinnamat | 3,00 | 3,60 | | 2,50 |
| Octocrylene | | | 7,50 | |
| Benzophenone-3 | | | 3,50 | |
| Tocopheryl Acetat | 0,50 | 1,00 | | |

(fortgesetzt)

| | 1 | 2 | 3 | 4 |
|---|---|---|---|---|
| Ascorbyl Palmitat | 0,05 | | 0,05 | |
| Buxus Chinensis | 2,00 | 1,00 | | 1,00 |
| Parfum, BHT | 0,10 | 0,25 | | 0,35 |
| Ricinus Communis | ad 100 | ad 100 | ad 100 | ad 100 |

**Patentansprüche**

1. Verwendung von öllöslichen und/oder in Ölen dispergierbaren Polyoxyethylen-Polydodecylglykol-Blockpolymeren zur Verbesserung der Wasserfestigkeit von O/W-Formulierungen.

2. Verwendung nach Anspruch 1, **dadurch gekennzeichnet, dass** die O/W-Formulierung eine Emulsion oder eine Hydrodispersion ist.

3. Verwendung nach einem der Ansprüche 1 oder 2, **dadurch gekennzeichnet, dass** die O/W-Formulierung eine Mikroemulsion ist.

4. Verwendung nach einem der vorhergehenden Ansprüche, **dadurch gekennzeichnet, dass** es sich bei den O/W-Formulierungen um kosmetische oder dermatologische Lichtschutzzubereitungen handelt, die als UV-A-Filtersubstanz ein oder mehrere wasserlösliche UV-A-Filtersubstanzen, besonders wasserlösliche UV-A-Filtersubstanzen gewählt aus der Gruppe Phenylen-1,4-bis-(2-benzimidazyl)-3,3'-5,5'-tetrasulfonsäure und/oder 1,4-di(2-oxo-10-Sulfo-3-bornylidenmethyl)-Benzol und/oder ihre Salze, besonders die entsprechenden Natrium-, Kalium- oder Triethanolammonium-Salze und/oder die entprechenden 10-Sulfato-verbindungen, insbesondere das Phenylen-1,4-bis-(2-benzimidazyl)-3,3'-5,5'-tetrasul-fonsäure-bis-natriumsalz, enthalten.

5. Verwendung nach einem der vorhergehenden Ansprüche, **dadurch gekennzeichnet, dass** es sich bei den O/W-Formulierungen um kosmetische oder dermatologische Lichtschutzzubereitungen handelt, die mindestens eine UV-Filtersubstanz, gewählt aus der folgenden Gruppe, enthält:

   wasserlösliche UV-Filtersubstanzen, bei Raumtemperatur flüssigen UV-Filter, organische und/oder anorganische Pigmente, Breitbandfilter (wie Bis-Resorcinyltriazinderivate, insbesondere das 2,4-Bis-{[4-(2-Ethyl-hexyloxy)-2-hydroxy]-phenyl}-6-(4-methoxyphenyl)-1,3,5-triazin) und/oder Benzotriazole.

6. Verwendung nach einem der vorhergehenden Ansprüche, **dadurch gekennzeichnet, dass** es sich bei den O/W-Formulierungen um kosmetische oder dermatologische Lichtschutzzubereitungen handelt, die mindestens eine UV-Filtersubstanz, gewählt aus der Benzoxazol-Derivate (insbesondere das 2,4-bis-[5-1 (dimethylpropyl)benzoxazol-2-yl-(4-phenyl)-imino]-6-(2-ethylhexyl)-imino-1,3,5-triazin und/oder aus der Gruppe der Hydroxybenzophenone (insbesondere der 2-(4'-Diethylamino-2'-hydoxybenzoyl)-benzoesäurehexylester) enthält.

7. Verwendung nach mindestens einem der vorhergehenden Ansprüche, **dadurch gekennzeichnet, dass** das Polyoxyethylen-Polydodecylglykol-Blockpolymer das PEG-45 Dodecyl Glycol Copolymer ist.

8. Verwendung nach mindestens einem der vorhergehenden Anprüche, **dadurch gekennzeichnet, dass** weitere Substanzen enthalten sind, die die Wasserfestigkeit der Zubereitungen erhöhen, insbesondere Substanzen gewählt aus der Gruppe, welche gebildet wird aus:

   - wasserlöslichen oder in Wasser dispergierbaren Polyoxyethylen-Polyoxypropylen-Blockpolymeren,
   - acetyliertem Glykolstearat mit Tristearin (INCI: Acetylated Glycol Stearat and Tristearin),
   - $C_{18-36}$ Fettsäuretriglycerid (INCI: C18-36 Acid Triglyceride) sowie
   - der unter der INCI-Bezeichnung C30-38 Olefin/Isopropyl Maleate/MA Copolymer bekannten Substanz.

## Claims

1. Use of oil-soluble and/or oil-dispersible polyoxyethylene-polydodecyl glycol block polymers for improving the water resistance of O/W formulations.

2. Use according to Claim 1, **characterized in that** the O/W formulation is an emulsion or a hydrodispersion.

3. Use according to either of Claims 1 and 2, **characterized in that** the O/W formulation is a microemulsion.

4. Use according to one of the preceding claims, **characterized in that** the O/W formulations are cosmetic or dermatological light protection preparations which comprise, as UV-A filter substance, one or more water-soluble UV-A filter substances, particularly water-soluble UV-A filter substances chosen from the group consisting of phenylene-1,4-bis(2-benzimidazyl)-3,3'-5,5'-tetrasulfonic acid and/or 1,4-di(2-oxo-10-sulfo-3-bornylidenemethyl)benzene and/or its salts, particularly the corresponding sodium, potassium or triethanolammonium salts and/or the corresponding 10-sulfato compounds, in particular the phenylene-1,4-bis(2-benzimidazyl)-3,3'-5,5'-tetrasulfonic acid bis-sodium salt.

5. Use according to any of the preceding claims, **characterized in that** the O/W formulations are cosmetic or dermatological light protection preparations which comprise at least one UV filter substance chosen from the following group:

   water-soluble UV filter substances, UV filters which are liquid at room temperature, organic and/or inorganic pigments, broadband filters (such as bis-resorcinyltriazine derivatives, in particular 2,4-bis-{[4-(2-ethylhexyloxy)-2-hydroxy]phenyl)-6-(4-methoxyphenyl)-1,3,5-triazine) and/or benzotriazoles.

6. Use according to any of the preceding claims, **characterized in that** the O/W formulations are cosmetic or dermatological light protection preparations which comprise at least one UV filter substance chosen from the benzoxazole derivatives (in particular the 2,4-bis[5-1-(dimethylpropyl)benzoxazol-2-yl(4-phenyl)imino]-6-(2-ethylhexyl)imino-1,3,5-triazine and/or from the group of hydroxybenzophenones (in particular hexyl 2-(4'-diethylamino-2'-hydroxybenzoyl)benzoate).

7. Use according to at least one of the preceding claims, **characterized in that** the polyoxyethylene-polydodecyl glycol block polymer is the PEG-45 dodecyl glycol copolymer.

8. Use according to at least one of the preceding claims, **characterized in that** further substances are present which increase the water resistance of the preparations, in particular substances chosen from the group which is formed from:

   • water-soluble or water-dispersible polyoxyethylene-polyoxypropylene block polymers,
   • acetylated glycol stearate with tristearin (INCI: **Acetylated** Glycol Stearate and Tristearin),
   • $C_{18-36}$ fatty acid triglyceride (INCI: C18-36 Acid Triglyceride) and
   • the substance known under the INCI name C30-38 Olefin/Isopropyl Maleate/MA Copolymer.

## Revendications

1. Utilisation de polymères séquencés polyoxyéthylène-polydodécylglycol liposolubles et/ou dispersables dans des huiles, pour l'amélioration de la résistance à l'eau de compositions H/E.

2. Utilisation selon la revendication 1, **caractérisée en ce que** la compositions H/E est une émulsion ou une hydrodispersion.

3. Utilisation selon l'une quelconque des revendications 1 et 2, **caractérisée en ce que** la compositions H/E est une microémulsion.

4. Utilisation selon l'une quelconque des revendications précédentes, **caractérisée en ce que** pour ce qui est des compositions H/E il s'agit de préparations photoprotectrices cosmétiques ou dermatologiques qui contiennent comme substance filtre UV-A une ou plusieurs substances filtres UV-A hydrosolubles, en particulier substances filtres UV-A hydrosolubles choisies dans le groupe constitué par l'acide phénylène-1,4-bis-(2-benzimidazyl)-3,3',-5,5',-tétrasulfonique et/ou le 1,4-di(2-oxo-10-sulfo-3-bornylidèneméthyl)-benzène et/ou leurs sels, en particulier les sels de sodium, potassium ou triéthanolammonium correspondants et/ou les composés 10-sulfato correspondants, en particulier le sel disodique d'acide phénylène-1,4-bis-(2-benzimidazyl)-3,3'-5,5'-tétrasulfonique.

**5.** Utilisation selon l'une quelconque des revendications précédentes, **caractérisée en ce que** pour ce qui est des compositions H/E il s'agit de préparations photoprotectrices cosmétiques ou dermatologiques qui contiennent au moins une substance filtre UV choisie dans le groupe suivant:

substances filtres UV hydrosolubles, filtres UV liquides à la température ambiant, pigments organiques et/ou inorganiques, filtres à large bande (tels que des dérivés de bis-résorcinyltriazine, en particulier la 2,4-bis-{[4-(2-éthyl-hexyloxy)-2-hydroxy]-phenyl}-6-(4-methoxyphényl)-1,3,5-triazine) et/ou des benzotriazoles.

**6.** Utilisation selon l'une quelconque des revendications précédentes, **caractérisée en ce que** pour ce qui est des compositions H/E il s'agit de préparations photoprotectrices cosmétiques ou dermatologiques qui contiennent au moins une substance filtre UV choisie parmi les dérivés de benzoxazole (en particulier la 2,4-bis-[5-1(diméthylpropyl)benzoxazol-2-yl-(4-phényl)-imino]-6-(2-éthylhexyl)-imino-1,3,5-triazine) et/ou dans le groupe des hydroxybenzophénones (en particulier le 2-(4'-diéthylamino-2'-hydroxy-benzoyl)-benzoate d'hexyle).

**7.** Utilisation selon au moins l'une quelconque des revendications précédentes, **caractérisée en ce que** le polymère séquencé polyoxyéthyléne-polydodécylglycol est le copolymère PEG-45 dodécylglycol.

**8.** Utilisation selon au moins l'une quelconque des revendications précédentes, **caractérisée en ce que** sont contenues d'autres substances qui augmentent la résistante à l'eau des préparations, en particulier des substances choisies dans le groupe qui est constitué par:

- des polymères séquences polyoxyéthylène-polyoxypropylène hydrosolubles ou dispersables dans l'eau,
- le stéarate de glycol acétylé avec la tristéarine (INCl: Acetylated Glycol Stearat and Tristearin),
- un triglycéride d'acide gras en $C_{18-36}$ (INCl: $C_{18-36}$ Acid Triglyceride) ainsi que
- la substance connue sous la dénomination INCI C30-38 Olefin/Isopropyl Maleate/MA Copolymer.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5663213 A
- EP 0761201 A